# FASCICULE DE BREVET EUROPEEN

(11) **EP 1 649 058 B1**
(45) Date de publication et mention de la délivrance du brevet: **20.10.2010**
(21) Numéro de dépôt: 04767753.9
(22) Date de dépôt: 22.07.2004
(51) Int. Cl.: C12Q 1/68

(54) **UTILISATION DU GENE SILVER POUR L'AUTHENTIFICATION DE L'ORIGINE RACIALE DES POPULATIONS ANIMALES ET DE LEURS PRODUITS DERIVES**
VERWENDUNG DES SILBER-GENS ZUR BESTÄTIGUNG DER RASSENHERKUNFT VON TIERPOPULATIONEN UND DER DAVON ABGELEITETEN PRODUKTE
USE OF SILVER GENE FOR THE AUTHENTICATION OF THE RACIAL ORIGIN OF ANIMAL POPULATIONS, AND OF THE DERIVATIVE PRODUCTS THEREOF

(30) Priorité: 25.07.2003 FR 0309161
(43) Date de publication de la demande: 26.04.2006
(73) Titulaire: INSTITUT NATIONAL DE LA RECHERCHE AGRONOMIQUE, 75338 Paris Cedex 07 (FR); UNIVERSITE DE LIMOGES, F-87065 Limoges Cedex (FR)
(72) Inventeur: OULMOUDEN, Ahmad, F-87350 Panazol (FR); JULIEN, Raymond, F-87000 Limoges (FR); LAFORET, Marie-Pierre, F-87350 Panazol (FR); LEVEZIEL, Hubert, F-87000 Limoges (FR)
(74) Mandataire: Grosset-Fournier, Chantal Catherine
(86) Numéro de dépôt international: PCT/FR2004/001952
(87) Numéro de publication internationale: WO 2005/019473

(56) Documents cités:
- WO-A-98/54360
- FR-A- 2 779 153
- DATABASE EBI [Online] Melanocyte protein Pmel17 1 novembre 1997 (1997-11-01), XP002275379 Database accession no. Q06154 -& KIM R.Y. ET AL.: "The cDNA RPE1 and monoclonal antibody HMB-50 define gene products preferentially expressed in retinal pigment epithelium" EXPERIMENTAL EYE RESEARCH, vol. 55, 1992, pages 657-662, XP009028579
- DATABASE EBI [Online] Bovine retinal pigment (RPE1) mRNA, 3' end 9 décembre 2001 (2001-12-09), XP002275380 Database accession no. M81193 -& KIM R.Y. ET AL.: "The cDNA RPE1 and monoclonal antibody HMB-50 define gene products preferentially expressed in retinal pigment epithelium" EXPERIMENTAL EYE RESEARCH, vol. 55, 1992, pages 657-662, XP009028579
- KOBAYASHI TAKESHI ET AL: "The Pmel 17/silver locus protein: Characterization and investigation of its melanogenic function" JOURNAL OF BIOLOGICAL CHEMISTRY, vol. 269, no. 46, 1994, pages 29198-29205, XP002275378 ISSN: 0021-9258
- ROUZAUD FRANCOIS ET AL: "A first genotyping assay of French cattle breeds based on a new allele of the extension gene encoding the melanocortin-1 receptor (Mc1r)" GENETICS SELECTION EVOLUTION (PARIS), vol. 32, no. 5, septembre 2000 (2000-09), pages 511-520, XP009028581 ISSN: 0999-193X
- DATABASE EBI [Online] CH240_373K8.TARBAC13P2 CHORI-240 Bos taurus 18 juin 2003 (2003-06-18), HOLT R. ET AL.: "Bovine BAC end sequences from library CHORI-240,plates 294-398" XP002275383 Database accession no. CC524467

### LISTE DE SEQUENCES

<110> INSTITUT NATIONAL DE LA RECHERCHE AGRONOMIQUE
<120> UTILISATION DU GENE SILVER COMME MARQUEUR
<130> IFB 03 BT INR SILV
<160> 11
<170> PatentIn version 3.1
<210> 1
   <211> 8146
   <212> ADN
   <213> Bos taurus
<220>
   <221> CDS
   <222> (30)..(105)
   <223>
<220>
   <221> CDS
   <222> (2326)..(2936)
   <223>
<220>
   <221> CDS
   <222> (2582)..(2728)
   <223>
<220>
   <221> CDS
   <222> (3804)..(3938)
   <223>
<220>
   <221> CDS
   <222> (4315)..(4476)
   <223>
<220>
   <221> CDS
   <222> (4733)..(5413)
   <223>
<220>
   <221> CDS
   <222> (6321)..(6937)
   <223>
<220>
   <221> CDS
   <222> (6681)..(6765)
   <223>
<220>
   <221> CDS
   <222> (6875)..(7080)
   <223>
<220>
   <221> CDS
   <222> (7188)..(7275)
   <223>
<220>
   <221> CDS
   <222> (7898)..(8036)
   <223>
<400> 1
   ggtctttggt tgctggaagg aagaacagg atg gat ctg gtg ctg aga aaa tac       53
                                   Met Asp Leu Val Leu Arg Lys Tyr
                                   1               5
   ctt ctc cat gtg gct ctg atg ggt gtt ctt ctg gct gta ggg acc aca      101
   Leu Leu His Val Ala Leu Met Gly Val Leu Leu Ala Val Gly Thr Thr
       10                  15                  20
   gaa g gtgagtgtgg gatgttggac atgaacaagt gtgaatttgg ggttgcacac         155
   Glu
   25
   ctgctctggt ttttctctcc ctaaaatgga agatatcagt agtgcttcag gtgtctccca   215
   cccatttgat ttagtgagga catgggcaac tgagctccct ccccacatga agatttgggt   275
   gcatgtgtgt tcaggcactt gggactgaac ctgaaaacaa ccccatctac ctggatgggt   335
   gagagaacag tatgtctccg tggccctaat tttgagatgc tctgaatagt gagctggaac   395
   atgggtgcca aggtagtaaa atgagtggaa actcatttag gctttgtctc aggcacttgg   455
   gatagggtat ttaggagata gagaaagata ggagatagga gaaaggagaa agaggatgtg   515
   gtattggata gaagggtaat gaggcacctc atcccctctt tgggatgggc atgggtgaac   575
   acagcccagg cttttgttct ggggctggaa gagacaggca gaagggtctc agctgagcat   635
   cacatgaaag ggctctgggg gattggggcc tcgtgacagg agcaaggcgg gtggggtggg   695
   gatggtgaga gggtctggaa tgtcccgtgc tgctctgagg agggaggatt gggagtggag   755
   aaagaatggg gcatcttatg attctcttgt tcttgtggtg aggtattcag tgggataatt   815
   ctagatcctc ccccaagaga atcaaccagg tttctggtac atgttagaga tggagtgagg   875
   atagtctgtg atgtgcagaa atatctacat tgtaccccag tgcccccttt ctctagatcc   935
   ctggtctcac agacttcttg gaacttctcc ttgatctgac ttccctcatt catggtgtca   995
   tttcaagtct tattctttta ctatgttcgt tattgtattc tggaaatatc ctgttcatat  1055
   gtgtccaccc aaggctctta atatgttgtg cttacttttt ggatccagat ttttaaaatc  1115
   ataagaagac atttttatat agttcatgaa attttgcatg gactgagttt gataattttg  1175
   tttagtgtga attaacattg tgtttattta agaaaaaaaa atattttttt acagaaacct  1235
   actgaatttg tagggtttta aaataacatg atgtctggga tttgcttttg aatgcttcag  1295
   ccaaaaaaca aacgaacaac aaaaataaag gatagataaa gcaaatgtga caaaatgctg  1355
   atagttgttg gaccttgggg agacacatgc agagccatca catcactttt tttcagacat  1415
   ctttcttggt cagttataat cattttgttt gtccccactc ccaatttcta cttgcctcta  1475
   gtccatcctc ctcactgctt gccaaagtga tccttctaaa acacaaatct gatcatattc  1535
   aaaaagcttt tgaagggtaa gttttatggt atatgccata tatcagtaca acaaacaaaa  1595
   tcgtctgagg tgccgttgcc tacaggataa agtccaaact cctttgcctg gcactccaag  1655
   cccccactct atcttcttgg cctcatctct catgatgtac atcagccaca ttgctagtgt  1715
   ctgctcatgg ccttctgcct agaatgcttt atgccccagc caactattta ctgtcttctt  1775
   cagtcgacca gagtgcaatt tacctgttta aaatctatca ttttgttata cattgtgcat  1835
   gtctattatg gctcatatta agcaatgcct tggattatag taatttatgt atatgtctat  1895
   ttcatatact ttaacctgaa ccccttcaga accatttctt tttcatttct taagttcttt  1955
   gcacctagcc cagtgcctgg tacgtcgtgg gtattcagta gattaaaatg cactttaagg  2015
   aacttccctt gttgtccatc aagtggctaa ggctctgtgc tcccaatgca ggggaccagg  2075
   gttcaatctc aggtcaggga actagatccc acaggtcaca actaagagtt tgcaagccac  2135
   aactacctga cctcacatgc cacaactaat cgaagatccc tcgtgctgca actaagtcct  2195
   agtgcagtta aatatatttt tttaatgcac tttgaatgtg agaatgaatg atgtgtcaca  2255
   gacactgttg tcccctgaga agggagtgag taatgatttg agggccctca tagtatatct  2315
   tcctttttag ga  ccc aga gac agg gac tgg ctt ggt gtc tca agg cag       2363
                  Gly Pro Arg Asp Arg Asp Trp Leu Gly Val Ser Arg Gln
                           30                  35
   ctc aga att aaa gca tgg aac aga cag ctg tat cca gag tgg aca gaa      2411
   Leu Arg Ile Lys Ala Trp Asn Arg Gln Leu Tyr Pro Glu Trp Thr Glu
       40                  45                  50
   agc cag ggg cct gac tgc tgg aga g gtaggaactt ggcaatttcc              2456
   Ser Gln Gly Pro Asp Cys Trp Arg
   55                  60
   agggaggata tggtggaaat gggtggggag gggaacgggg ttgaatgtac ttaggaagat   2516
   agggaaggaa aaggcataca gggaggagaa gccaaggagc taattaatgc agctgccctt   2576
   ttcag gt  ggc cac ata tcc ctg aag gtc agc aat gat ggg cct aca ctg   2625
             Gly Gly His Ile Ser Leu Lys Val Ser Asn Asp Gly Pro Thr Leu
                      65                  70                  75
   att ggg gca aat gct tcc ttc tct att gcc ttg cac ttt cct aaa agc      2673
   Ile Gly Ala Asn Ala Ser Phe Ser Ile Ala Leu His Phe Pro Lys Ser
           80                  85                  90
   caa aag gtg ctg cca gat ggg cag gtc atc tgg gcc aac aac acc atc      2721
   Gln Lys Val Leu Pro Asp Gly Gln Val Ile Trp Ala Asn Asn Thr Ile
       95                  100                 105
   atc aat g gtgagtacct ctccgcctcc ttcccaaggt ccagaatccc tggtatcccc    2778
   Ile Asn
   110
   aatgagctca aggaatcctc ctcctctttt tttttttttt tttttacaaa ttatatatgt   2838
   aacacatatt cactgcagaa aaattagaaa acacagataa accaaaaaga aaaaaaatta   2898
   tagttcccca aatggggcac agaagaccca gtggacatag aagttggata gacttggatt   2958
   taaactggtt accagtatgt gaccctggac aagtcactga attgttttgt tcttccattc   3018
   ccttatctat agaatgggga tgataacact ttaaaaggtt cttgtaagga ttaaaatgtg   3078
   ataatatata aagattttag cataatgcct gccctgtgct gtgcttagta ccttagttta   3138
   gacgctttgc aaccccatgg actgtagccc accaggctcc tctgtccatg tggattctgc   3198
   aggcaagaat actggagtgg gtcaccatgc actcctccag gggatcttcc caactcaggg   3258
   atcgaaccca ggtcctagcc tacagtatta attgatgctg ttatttttac ttttatccca   3318
   ctagctagag cacatcatcc tagacatttt gatacatggc ctaccaattt gtgtccagtg   3378
   taagaatata catgtgtgtg ctcagtggct cagtcgtgtc tgactctttg caaccccatg   3438
   gactgtagcc cgcgaaagct cctctgccca tgggattgcc cagccaagaa tactggagca   3498
   ggttgccatt tcttcctcca ggggatcttt caacacaggg attgaatcct tgtctcctgt   3558
   gtttcctgca ttggcaggtg tattctttac cactgagcca cctgggaaac cccttaagta   3618
   tatacacata aatcttttat agtttccatt ctcccttcta ccactccaaa taggttatac   3678
   caaggagaat gtattttggt agctaggcag tattcctgga gcccctctct gggagtcatg   3738
   ttaaaggttt tggtgtacag tgaggaatgc cagggattga gggagacttg ctgtcttctt   3798
   ttcag gg  agc cag gtg tgg gga gga cag ctg gta tat ccc caa gaa cct   3847
             Gly Ser Gln Val Trp Gly Gly Gln Leu Val Tyr Pro Gln Glu Pro
                         115                 120                 125
   gat gat acc tgc atc ttc ccc gat ggg gag ccc tgc cct tct ggc cct      3895
   Asp Asp Thr Cys Ile Phe Pro Asp Gly Glu Pro Cys Pro Ser Gly Pro
                   130                 135                 140
   cta tct cag aaa aga tgc ttt gtt tat gtc tgg aag acc tgg g            3938
   Leu Ser Gln Lys Arg Cys Phe Val Tyr Val Trp Lys Thr Trp
           145                 150                 155
   gtaagagttt cccttctctg gcctgtcatt cacacttaaa ttcacttctt cctacctgat   3998
   cccctttctt ttggtctcat ccttaaattc tgtgagtttc cctaatcttc acttccccca  4058
   tgactccttc ctcttccaca gcacctagtc aactctatta tacttctttc tgggagccct   4118
   gctccaatta tagtcccatc ccatggaccc tctcataagg acttttttcc tgcccaacat  4178
   atgcaagctt aaactctctg aaataaccat ccttgataca tctcctgacc ttccttctct   4238
   ggttccatct ctaaccctgc cccagtctcc tttgaccagt aacccccttc cctactcttc  4298
   tttccaaaaa cctcag ac  caa tac tgg caa gtt ctg ggg ggc cca gtg tct   4349
                      Asp Gln Tyr Trp Gln Val Leu Gly Gly Pro Val Ser
                                      160                 165
   gga ctg agc atc ggg aca gac aag gca atg ctg ggc aca tat aac atg     4397
   Gly Leu Ser Ile Gly Thr Asp Lys Ala Met Leu Gly Thr Tyr Asn Met
       170                 175                 180
   gaa gtg act gtc tac cac cgc cgg ggg tcc cag agc tat gtg ccc ctc     4445
   Glu Val Thr Val Tyr His Arg Arg Gly Ser Gln Ser Tyr Val Pro Leu
   185                 190                 195                 200
   gct cac tcc agt tca gcc ttc acc att act g gtaaggactg aggaggggac     4496
   Ala His Ser Ser Ser Ala Phe Thr Ile Thr
                   205                 210
   aaggccagtt gcagggcagg agaaggtggg gaggctgggc tggacaggaa aggggaaaga   4556
   ggaaatggtg tgtaacctta caggggcaga accaggaaga tgtgggcaga gggatgtggg   4616
   gcttggagcc cgtgaagggc caggcagctt gggttggttg aaaaatatgg ctgtgaaaga   4676
   agaagctgac agaaagaaga acttatggtt ctcactttct ctgactccaa tcccag ac    4734
                                                                 Asp
   cag gtg ccc ttc tct gtg agt gtg tct cag ctg cag gcc ttg gat gga     4782
   Gln Val Pro Phe Ser Val Ser Val Ser Gln Leu Gln Ala Leu Asp Gly
               215                 220                 225
   agg aac aag cgc ttc ctg aga aag cag cct ctg acc ttt gcc ctc cag     4830
   Arg Asn Lys Arg Phe Leu Arg Lys Gln Pro Leu Thr Phe Ala Leu Gln
           230                 235                 240
   ctc cat gat ccc agt ggc tat ttg gct ggg gct gac ctt tcc tac acc     4878
   Leu His Asp Pro Ser Gly Tyr Leu Ala Gly Ala Asp Leu Ser Tyr Thr
       245                 250                 255
   tgg gac ttt ggt gac agt aca ggg acc ctg atc tct cgg gca ctc acg     4926
   Trp Asp Phe Gly Asp Ser Thr Gly Thr Leu Ile Ser Arg Ala Leu Thr
   260                 265                 270                 275
   gtc act cac act tac cta gag tct ggc cca gtc act gca cag gtg gtg     4974
   Val Thr His Thr Tyr Leu Glu Ser Gly Pro Val Thr Ala Gln Val Val
                   280                 285                 290
   ctg cag gct gcc att cct ctc acc tcc tgt ggc tcc tct cca gtt cca     5022
   Leu Gln Ala Ala Ile Pro Leu Thr Ser Cys Gly Ser Ser Pro Val Pro
               295                 300                 305
   ggc act aca gat agg cat gtg aca act gca gag gct cct gga acc aca     5070
   Gly Thr Thr Asp Arg His Val Thr Thr Ala Glu Ala Pro Gly Thr Thr
           310                 315                 320
   gct ggc caa gtg cct act aca gaa gtc atg ggc acc aca cct ggc cag      5118
   Ala Gly Gln Val Pro Thr Thr Glu Val Met Gly Thr Thr Pro Gly Gln
       325                 330                 335
   gtg cca act gca gag gcc cct ggc acc aca gtt ggg tgg gtg cca acc      5166
   Val Pro Thr Ala Glu Ala Pro Gly Thr Thr Val Gly Trp Val Pro Thr
   340                 345                 350                 355
   aca gag gat gta ggt acc aca cct gag cag gtg gca acc tcc aaa gtc      5214
   Thr Glu Asp Val Gly Thr Thr Pro Glu Gln Val Ala Thr Ser Lys Val
                   360                 365                 370
   tta agt aca aca cca gtg gag atg cca act gca aaa gct aca ggt agg      5262
   Leu Ser Thr Thr Pro Val Glu Met Pro Thr Ala Lys Ala Thr Gly Arg
               375                 380                 385
   aca cct gaa gtg tca act aca gag ccc tct gga acc aca gtt aca cag      5310
   Thr Pro Glu Val Ser Thr Thr Glu Pro Ser Gly Thr Thr Val Thr Gln
           390                 395                 400
   gga aca act cca gag ctg gtg gag acc aca gct gga gag gtg tcc act      5358
   Gly Thr Thr Pro Glu Leu Val Glu Thr Thr Ala Gly Glu Val Ser Thr
       405                 410                 415
   cct gag cct gcg ggt tca aat act agc tca ttc atg cct aca gaa ggt      5406
   Pro Glu Pro Ala Gly Ser Asn Thr Ser Ser Phe Met Pro Thr Glu Gly
   420                 425                 430                 435
   act gca g gtaagggggc caccatgaat gagttcatag aggtggggca tttgtcacag    5463
   Thr Ala
   ctctgaagac ctgaaagaat tgctcaggac ccagatgtta ctcaatcctt agcttagcag    5523
   tggagtcccc tcagaatctt cactggtttt aaaaccccct aagtccctct taatggcaca     5583
   gaatagatcc agagttcagg aaaccagggt cttctcctag gccaggggta gagagcttat    5643
   tctctcttcc tgaagagaag ttcaggaagc agtgtgtgat catttggtgg tggtgctcag    5703
   tcatgtctga ctctttgtga cctcatggac tatggcccac caggctcctc tgtccataga    5763
   attctccagg caagaacact ggagtgggtg gccatttcct tctccagggg attttccctg    5823
   cccagggatt aaacccgaat tggcaggtgg attctttacc cgagccacct agaaagtccc    5883
   atgtgatcat tagataatac ttatacctca ttttctgatt aagtgtaaac acagaaatct    5943
   ttctgacacc acttcccacc cctggattcc catcccaaag taggtttacc tggaattgtg    6003
   gtaggaatac taaaaaggga gaagtgagat agtgacacta tgacttaaca catgtcaaat    6063
   gtctgaccca ggacctggca cagtgtaggg tgtgataaac atttgggatg tctaaaattc    6123
   tgactctaac cctgtgactc tggggcagtc atttctcttg ggcctttctt tatcttaaaa    6183
   aatgagagtt tccagctctt gtctgattct aagcctggat ccagtagctc tgactctacc    6243
   tggaaaaatg cttgttgggc ctgttttcag gttagtcatt tgctttttga ctttgcctct    6303
   ttaatcctct cctccag gc  tcc ctg agt ccc ctg ccg gat gac act gcc       6352
                       Gly Ser Leu Ser Pro Leu Pro Asp Asp Thr Ala
                               440                 445
   acc tta gtc ctg gag aag cgc caa gcc ccc ctg gat tgt gtt ctg tat      6400
   Thr Leu Val Leu Glu Lys Arg Gln Ala Pro Leu Asp Cys Val Leu Tyr
       450                 455                 460
   cgc tat ggc tcc ttt tcc ctc acc ctg gac att gtc c gtgagtcttg         6447
   Arg Tyr Gly Ser Phe Ser Leu Thr Leu Asp Ile Val
   465                 470                 475
   cctacattgt ccgtaagctg gtggagggag gcgtgtgctg cttagggttg cccagtggaa    6507
   gcacaccttg gaaggaatta ctcacctgga caaggagaat acccagatcc caggggtttc    6567
   atatgaaggc agaatgggat tagggaggca gcccgaggac cttcctggcc atgggccttg   6627
   ggggaggata agtagaggag tctcagactt aaaaaaatct tgcaactttg cag ag       6682
                                                               Gln
   ggt att gag agt gct gag atc cta cag gct gtg tca tcc agt gaa gga     6730
   Gly Ile Glu Ser Ala Glu Ile Leu Gln Ala Val Ser Ser Ser Glu Gly
           480                 485                 490
   gat gca ttt gag ctg act gtg tct tgc caa ggc gg  gtgagtgtcc          6775
   Asp Ala Phe Glu Leu Thr Val Ser Cys Gln Gly Gly
       495                 500
   cacggttgcc ctgagaactc ctggggtgac tgctgtcctg ttctctggtg tctagtgtcc   6835
   cttcccagat tccctgacgt aagctgacat ctctcccag g cta ccc aag gaa gcc    6890
                                                 Leu Pro Lys Glu Ala
                                                                 510
   tgc atg gac atc tca tcg cca ggg tgt cag ctg cct gcc cag cgg ctg     6938
   Cys Met Asp Ile Ser Ser Pro Gly Cys Gln Leu Pro Ala Gln Arg Leu
                   515                 520                 525
   tgt cag cct gtg ccc ccc agc cca gcc tgc cag ctg gtt ttg cac cag     6986
   Cys Gln Pro Val Pro Pro Ser Pro Ala Cys Gln Leu Val Leu His Gln
               530                 535                 540
   gta ctg aag ggt ggc tca ggg acc tac tgc ctc aat gtg tct ttg gct     7034
   Val Leu Lys Gly Gly Ser Gly Thr Tyr Cys Leu Asn Val Ser Leu Ala
           545                 550                 555
   gat gcc aat agc ctg gcg atg gtc agc acc cag ctt gtc atg cct g       7080
   Asp Ala Asn Ser Leu Ala Met Val Ser Thr Gln Leu Val Met Pro
       560                 565                 570
   gtaggtagtt ggacaagagg taggatgaag acacggggag atggtagagg ttacctacta   7140
   gaggaagcag acactgaatg cagccgtatc tgggattcca cccatag gg  caa gaa     7195
                                                    Gly Gln Glu
                                                        575
   gca ggc ctc agg cag gct cct ctg ttc gtg ggc atc ttg ctg gtg cta     7243
   Ala Gly Leu Arg Gln Ala Pro Leu Phe Val Gly Ile Leu Leu Val Leu
               580                 585                 590
   aca gct ttg ttg ctt gca tct ctg ata tac ag  gtgagatccc cgccatcctg   7295
   Thr Ala Leu Leu Leu Ala Ser Leu Ile Tyr Arg
           595                 600
   ctcccactcc tttacccctt attaccacca ccactcttcc tcatgggaag aagaaaccac   7355
   caaccccttt gggaaagtgt agagtccaag aaagagccca gacttggaag ttcaacaggt  7415
   ctaggctgca gtcttgctgg tgggaccctg gggaagtcca ttaacccttc tgagccactg   7475
   aaaagtagga aacataatac ctgtcctgtg gggctgtttt cagggctcta gacaatgtga    7535
   gtaaaacacc tggttctgaa acaaaagtgg aataaatgat gatctcaatg actgttgtta   7595
   tgaataatat caacagtgga gaagaactca gtgaactgag ttctccacct gccagaaagg   7655
   caaatcccta ggcctggagg gctgaggtcc tcaaagcagg gaagcctgta gggtgagagg   7715
   gaaatggtca gagcttacca taaacataag agaggataaa ccctgttggt gagaagagga   7775
   gggagccagg atcaagacca agtcaacctg ggttatggtt tagtcttttt tttttagaga   7835
   agcacaaaga ggttgccatt gaccaccact aaccagtatc cctgcttttc tcccaatatc   7895
   ag g cga aga ctt atg aag caa ggc tca gca gtc ccc ctt ccc cag ctg     7943
        Arg Arg Leu Met Lys Gln Gly Ser Ala Val Pro Leu Pro Gln Leu
            605                 610                 615
   cca cac ggt aga acc cag tgg cta cgt ctg ccc tgg gtc ttc cgc tct      7991
   Pro His Gly Arg Thr Gln Trp Leu Arg Leu Pro Trp Val Phe Arg Ser
       620                 625                 630
   tgc ccc att ggt gag agc aaa ccc ctc ctc agt gga cag cag gtc          8036
   Cys Pro Ile Gly Glu Ser Lys Pro Leu Leu Ser Gly Gln Gln Val
   635                 640                 645
   tgagtgctct tatgtgaagt catgatttac ccaggtggac agcaaggcct gtcttttctc    8096
   tggtcttccc tcagagacta ccattgcctg aaataaagac tcagaacttg               8146
<210> 3
   <211> 8146
   <212> ADN
   <213> Bos taurus
<220>
   <221> CDS
   <222> (30)..(105)
   <223>
<220>
   <221> CDS
   <222> (2326)..(2436)
   <223>
<220>
   <221> CDS
   <222> (2582)..(2728)
   <223>
<220>
   <221> CDS
   <222> (3804)..(3938)
   <223>
<220>
   <221> CDS
   <222> (4315)..(4476)
   <223>
<220>
   <221> CDS
   <222> (4733)..(5413)
   <223>
<220>
   <221> CDS
   <222> (6321)..(6437)
   <223>
<220>
   <221> CDS
   <222> (6681)..(6765)
   <223>
<220>
   <221> CDS
   <222> (6875)..(7080)
   <223>
<220>
   <221> CDS
   <222> (7188)..(7275)
   <223>
<220>
   <221> CDS
   <222> (7898)..(8036)
   <223>
<400> 3
   ggtctttggt tgctggaagg aagaacagg atg gat ctg gtg ctg aga aaa tac       53
                                   Met Asp Leu Val Leu Arg Lys Tyr
                                   1               5
   ctt ctc cat gtg gct ctg atg ggt gtt ctt ctg gct gta agg acc aca      101
   Leu Leu His Val Ala Leu Met Gly Val Leu Leu Ala Val Arg Thr Thr
       10                  15                  20
   gaa g gtgagtgtgg gatgttggac atgaacaagt gtgaatttgg ggttgcacac         155
   Glu
   25
   ctgctctggt ttttctctcc ctaaaatgga agatatcagt agtgcttcag gtgtctccca    215
   cccatttgat ttagtgagga catgggcaac tgagctccct ccccacatga agatttgggt    275
   gcatgtgtgt tcaggcactt gggactgaac ctgaaaacaa ccccatctac ctggatgggt    335
   gagagaacag tatgtctccg tggccctaat tttgagatgc tctgaatagt gagctggaac    395
   atgggtgcca aggtagtaaa atgagtggaa actcatttag gctttgtctc aggcacttgg    455
   gatagggtat ttaggagata gagaaagata ggagatagga gaaaggagaa agaggatgtg    515
   gtattggata gaagggtaat gaggcacctc atcccctctt tgggatgggc atgggtgaac    575
   acagcccagg cttttgttct ggggctggaa gagacaggca gaagggtctc agctgagcat    635
   cacatgaaag ggctctgggg gattggggcc tcgtgacagg agcaaggcgg gtggggtggg    695
   gatggtgaga gggtctggaa tgtcccgtgc tgctctgagg agggaggatt gggagtggag    755
   aaagaatggg gcatcttatg attctcttgt tcttgtggtg aggtattcag tgggataatt    815
   ctagatcctc ccccaagaga atcaaccagg tttctggtac atgttagaga tggagtgagg    875
   atagtctgtg atgtgcagaa atatctacat tgtaccccag tgcccccttt ctctagatcc    935
   ctggtctcac agacttcttg gaacttctcc ttgatctgac ttccctcatt catggtgtca    995
   tttcaagtct tattctttta ctatgttcgt tattgtattc tggaaatatc ctgttcatat   1055
   gtgtccaccc aaggctctta atatgttgtg cttacttttt ggatccagat ttttaaaatc   1115
   ataagaagac atttttatat agttcatgaa attttgcatg gactgagttt gataattttg   1175
   tttagtgtga attaacattg tgtttattta agaaaaaaaa atattttttt acagaaacct   1235
   actgaatttg tagggtttta aaataacatg atgtctggga tttgcttttg aatgcttcag   1295
   ccaaaaaaca aacgaacaac aaaaataaag gatagataaa gcaaatgtga caaaatgctg   1355
   atagttgttg gaccttgggg agacacatgc agagccatca catcactttt tttcagacat   1415
   ctttcttggt cagttataat cattttgttt gtccccactc ccaatttcta cttgcctcta   1475
   gtccatcctc ctcactgctt gccaaagtga tccttctaaa acacaaatct gatcatattc   1535
   aaaaagcttt tgaagggtaa gttttatggt atatgccata tatcagtaca acaaacaaaa   1595
   tcgtctgagg tgccgttgcc tacaggataa agtccaaact cctttgcctg gcactccaag   1655
   cccccactct atcttcttgg cctcatctct catgatgtac atcagccaca ttgctagtgt   1715
   ctgctcatgg ccttctgcct agaatgcttt atgccccagc caactattta ctgtcttctt   1775
   cagtcgacca gagtgcaatt tacctgttta aaatctatca ttttgttata cattgtgcat   1835
   gtctattatg gctcatatta agcaatgcct tggattatag taatttatgt atatgtctat   1895
   ttcatatact ttaacctgaa ccccttcaga accatttctt tttcatttct taagttcttt   1955
   gcacctagcc cagtgcctgg tacgtcgtgg gtattcagta gattaaaatg cactttaagg   2015
   aacttccctt gttgtccatc aagtggctaa ggctctgtgc tcccaatgca ggggaccagg   2075
   gttcaatctc aggtcaggga actagatccc acaggtcaca actaagagtt tgcaagccac   2135
   aactacctga cctcacatgc cacaactaat cgaagatccc tcgtgctgca actaagtcct   2195
   agtgcagtta aatatatttt tttaatgcac tttgaatgtg agaatgaatg atgtgtcaca   2255
   gacactgttg tcccctgaga agggagtgag taatgatttg agggccctca tagtatatct   2315
   tcctttttag ga  ccc aga gac agg gac tgg ctt ggt gtc tca agg cag      2363
                  Gly Pro Arg Asp Arg Asp Trp Leu Gly Val Ser Arg Gln
                                   30                  35
   ctc aga att aaa gca tgg aac aga cag ctg tat cca gag tgg aca gaa    2411
   Leu Arg Ile Lys Ala Trp Asn Arg Gln Leu Tyr Pro Glu Trp Thr Glu
       40                  45                  50
   agc cag ggg cct gac tgc tgg aga g gtaggaactt ggcaatttcc            2456
   Ser Gln Gly Pro Asp Cys Trp Arg
   55                  60
   agggaggata tggtggaaat gggtggggag gggaacgggg ttgaatgtac ttaggaagat   2516
   agggaaggaa aaggcataca gggaggagaa gccaaggagc taattaatgc agctgccctt   2576
   ttcag gt  ggc cac ata tcc ctg aag gtc agc aat gat ggg cct aca ctg   2625
         Gly Gly His Ile Ser Leu Lys Val Ser Asn Asp Gly Pro Thr Leu
                 65                  70                  75
   att ggg gca aat gct tcc ttc tct att gcc ttg cac ttt cct aaa agc    2673
   Ile Gly Ala Asn Ala Ser Phe Ser Ile Ala Leu His Phe Pro Lys Ser
           80                  85                  90
   caa aag gtg ctg cca gat ggg cag gtc atc tgg gcc aac aac acc atc    2721
   Gln Lys Val Leu Pro Asp Gly Gln Val Ile Trp Ala Asn Asn Thr Ile
       95                  100                 105
   atc aat g gtgagtacct ctccgcctcc ttcccaaggt ccagaatccc tggtatcccc   2778
   Ile Asn
   110
   aatgagctca aggaatcctc ctcctctttt tttttttttt tttttacaaa ttatatatgt   2838
   aacacatatt cactgcagaa aaattagaaa acacagataa accaaaaaga aaaaaaatta   2898
   tagttcccca aatggggcac agaagaccca gtggacatag aagttggata gacttggatt   2958
   taaactggtt accagtatgt gaccctggac aagtcactga attgttttgt tcttccattc   3018
   ccttatctat agaatgggga tgataacact ttaaaaggtt cttgtaagga ttaaaatgtg   3078
   ataatatata aagattttag cataatgcct gccctgtgct gtgcttagta ccttagttta    3138
   gacgctttgc aaccccatgg actgtagccc accaggctcc tctgtccatg tggattctgc   3198
   aggcaagaat actggagtgg gtcaccatgc actcctccag gggatcttcc caactcaggg   3258
   atcgaaccca ggtcctagcc tacagtatta attgatgctg ttatttttac ttttatccca   3318
   ctagctagag cacatcatcc tagacatttt gatacatggc ctaccaattt gtgtccagtg   3378
   taagaatata catgtgtgtg ctcagtggct cagtcgtgtc tgactctttg caaccccatg   3438
   gactgtagcc cgcgaaagct cctctgccca tgggattgcc cagccaagaa tactggagca   3498
   ggttgccatt tcttcctcca ggggatcttt caacacaggg attgaatcct tgtctcctgt   3558
   gtttcctgca ttggcaggtg tattctttac cactgagcca cctgggaaac cccttaagta   3618
   tatacacata aatcttttat agtttccatt ctcccttcta ccactccaaa taggttatac   3678
   caaggagaat gtattttggt agctaggcag tattcctgga gcccctctct gggagtcatg   3738
   ttaaaggttt tggtgtacag tgaggaatgc cagggattga gggagacttg ctgtcttctt   3798
   ttcag gg  agc cag gtg tgg gga gga cag ctg gta tat ccc caa gaa cct   3847
         Gly Ser Gln Val Trp Gly Gly Gln Leu Val Tyr Pro Gln Glu Pro
                     115                 120                 125
   gat gat acc tgc atc ttc ccc gat ggg gag ccc tgc cct tct ggc cct     3895
   Asp Asp Thr Cys Ile Phe Pro Asp Gly Glu Pro Cys Pro Ser Gly Pro
               130                 135                 140
   cta tct cag aaa aga tgc ttt gtt tat gtc tgg aag acc tgg g           3938
   Leu Ser Gln Lys Arg Cys Phe Val Tyr Val Trp Lys Thr Trp
           145                 150                 155
   gtaagagttt cccttctctg gcctgtcatt cacacttaaa ttcacttctt cctacctgat   3998
   cccctttctt ttggtctcat ccttaaattc tgtgagtttc cctaatcttc acttccccca   4058
   tgactccttc ctcttccaca gcacctagtc aactctatta tacttctttc tgggagccct   4118
   gctccaatta tagtcccatc ccatggaccc tctcataagg acttttttcc tgcccaacat  4178
   atgcaagctt aaactctctg aaataaccat ccttgataca tctcctgacc ttccttctct   4238
   ggttccatct ctaaccctgc cccagtctcc tttgaccagt aacccccttc cctactcttc  4298
   tttccaaaaa cctca  gac caa tac tgg caa gtt ctg ggg ggc cca gtg tct   4349
                     Asp Gln Tyr Trp Gln Val Leu Gly Gly Pro Val Ser
                                 160                 165
   gga ctg agc atc ggg aca gac aag gca atg ctg ggc aca tat aac atg     4397
   Gly Leu Ser Ile Gly Thr Asp Lys Ala Met Leu Gly Thr Tyr Asn Met
       170                 175                 180
   gaa gtg act gtc tac cac cgc cgg ggg tcc cag agc tat gtg ccc ctc     4445
   Glu Val Thr Val Tyr His Arg Arg Gly Ser Gln Ser Tyr Val Pro Leu
   185                 190                 195                 200
   gct cac tcc agt tca gcc ttc acc att act g gtaaggactg aggaggggac     4496
   Ala His Ser Ser Ser Ala Phe Thr Ile Thr
                   205                 210
   aaggccagtt gcagggcagg agaaggtggg gaggctgggc tggacaggaa aggggaaaga   4556
   ggaaatggtg tgtaacctta caggggcaga accaggaaga tgtgggcaga gggatgtggg   4616
   gcttggagcc cgtgaagggc caggcagctt gggttggttg aaaaatatgg ctgtgaaaga   4676
   agaagctgac agaaagaaga acttatggtt ctcactttct ctgactccaa tccca gac     4734
                                                                Asp
   cag gtg ccc ttc tct gtg agt gtg tct cag ctg cag gcc ttg gat gga     4782
   Gln Val Pro Phe Ser Val Ser Val Ser Gln Leu Gln Ala Leu Asp Gly
               215                 220                 225
   agg aac aag cgc ttc ctg aga aag cag cct ctg acc ttt gcc ctc cag     4830
   Arg Asn Lys Arg Phe Leu Arg Lys Gln Pro Leu Thr Phe Ala Leu Gln
           230                 235                 240
   ctc cat gat ccc agt ggc tat ttg gct ggg gct gac ctt tcc tac acc     4878
   Leu His Asp Pro Ser Gly Tyr Leu Ala Gly Ala Asp Leu Ser Tyr Thr
       245                 250                 255
   tgg gac ttt ggt gac agt aca ggg acc ctg atc tct cgg gca ctc acg     4926
   Trp Asp Phe Gly Asp Ser Thr Gly Thr Leu Ile Ser Arg Ala Leu Thr
   260                 265                 270                 275
   gtc act cac act tac cta gag tct ggc cca gtc act gca cag gtg gtg      4974
   Val Thr His Thr Tyr Leu Glu Ser Gly Pro Val Thr Ala Gln Val Val
                   280                 285                 290
   ctg cag gct gcc att cct ctc acc tcc tgt ggc tcc tct cca gtt cca      5022
   Leu Gln Ala Ala Ile Pro Leu Thr Ser Cys Gly Ser Ser Pro Val Pro
               295                 300                 305
   ggc act aca gat agg cat gtg aca act gca gag gct cct gga acc aca      5070
   Gly Thr Thr Asp Arg His Val Thr Thr Ala Glu Ala Pro Gly Thr Thr
           310                 315                 320
   gct ggc caa gtg cct act aca gaa gtc atg ggc acc aca cct ggc cag      5118
   Ala Gly Gln Val Pro Thr Thr Glu Val Met Gly Thr Thr Pro Gly Gln
       325                 330                 335
   gtg cca act gca gag gcc cct ggc acc aca gtt ggg tgg gtg cca acc      5166
   Val Pro Thr Ala Glu Ala Pro Gly Thr Thr Val Gly Trp Val Pro Thr
   340                 345                 350                 355
   aca gag gat gta ggt acc aca cct gag cag gtg gca acc tcc aaa gtc      5214
   Thr Glu Asp Val Gly Thr Thr Pro Glu Gln Val Ala Thr Ser Lys Val
                   360                 365                 370
   tta agt aca aca cca gtg gag atg cca act gca aaa gct aca ggt agg      5262
   Leu Ser Thr Thr Pro Val Glu Met Pro Thr Ala Lys Ala Thr Gly Arg
               375                 380                 385
   aca cct gaa gtg tca act aca gag ccc tct gga acc aca gtt aca cag      5310
   Thr Pro Glu Val Ser Thr Thr Glu Pro Ser Gly Thr Thr Val Thr Gln
           390                 395                 400
   gga aca act cca gag ctg gtg gag acc aca gct gga gag gtg tcc act      5358
   Gly Thr Thr Pro Glu Leu Val Glu Thr Thr Ala Gly Glu Val Ser Thr
       405                 410                 415
   cct gag cct gcg ggt tca aat act agc tca ttc atg cct aca gaa ggt      5406
   Pro Glu Pro Ala Gly Ser Asn Thr Ser Ser Phe Met Pro Thr Glu Gly
   420                 425                 430                 435
   act gca g gtaagggggc caccatgaat gagttcatag aggtggggca tttgtcacag      5463
   Thr Ala
   ctctgaagac ctgaaagaat tgctcaggac ccagatgtta ctcaatcctt agcttagcag   5523
   tggagtcccc tcagaatctt cactggtttt aaaaccccct aagtccctct taatggcaca    5583
   gaatagatcc agagttcagg aaaccagggt cttctcctag gccaggggta gagagcttat   5643
   tctctcttcc tgaagagaag ttcaggaagc agtgtgtgat catttggtgg tggtgctcag   5703
   tcatgtctga ctctttgtga cctcatggac tatggcccac caggctcctc tgtccataga   5763
   attctccagg caagaacact ggagtgggtg gccatttcct tctccagggg attttccctg   5823
   cccagggatt aaacccgaat tggcaggtgg attctttacc cgagccacct agaaagtccc   5883
   atgtgatcat tagataatac ttatacctca ttttctgatt aagtgtaaac acagaaatct   5943
   ttctgacacc acttcccacc cctggattcc catcccaaag taggtttacc tggaattgtg   6003
   gtaggaatac taaaaaggga gaagtgagat agtgacacta tgacttaaca catgtcaaat   6063
   gtctgaccca ggacctggca cagtgtaggg tgtgataaac atttgggatg tctaaaattc   6123
   tgactctaac cctgtgactc tggggcagtc atttctcttg ggcctttctt tatcttaaaa   6183
   aatgagagtt tccagctctt gtctgattct aagcctggat ccagtagctc tgactctacc   6243
   tggaaaaatg cttgttgggc ctgttttcag gttagtcatt tgctttttga ctttgcctct   6303
   ttaatcctct cctccag gc  tcc ctg agt ccc ctg ccg gat gac act gcc      6352
                      Gly Ser Leu Ser Pro Leu Pro Asp Asp Thr Ala
                               440                 445
   acc tta gtc ctg gag aag cgc caa gcc ccc ctg gat tgt gtt ctg tat      6400
   Thr Leu Val Leu Glu Lys Arg Gln Ala Pro Leu Asp Cys Val Leu Tyr
       450                 455                 460
   cgc tat ggc tcc ttt tcc ctc acc ctg gac att gtc c gtgagtcttg         6447
   Arg Tyr Gly Ser Phe Ser Leu Thr Leu Asp Ile Val
   465                 470                 475
   cctacattgt ccgtaagctg gtggagggag gcgtgtgctg cttagggttg cccagtggaa   6507
   gcacaccttg gaaggaatta ctcacctgga caaggagaat acccagatcc caggggtttc   6567
   atatgaaggc agaatgggat tagggaggca gcccgaggac cttcctggcc atgggccttg   6627
   ggggaggata agtagaggag tctcagactt aaaaaaatct tgcaactttg cag ag        6682
                                                            Gln
   ggt att gag agt gct gag atc cta cag gct gtg tca tcc agt gaa gga      6730
   Gly Ile Glu Ser Ala Glu Ile Leu Gln Ala Val Ser Ser Ser Glu Gly
           480                 485                 490
   gat gca ttt gag ctg act gtg tct tgc caa ggc gg  gtgagtgtcc          6775
   Asp Ala Phe Glu Leu Thr Val Ser Cys Gln Gly Gly
       495                 500
   cacggttgcc ctgagaactc ctggggtgac tgctgtcctg ttctctggtg tctagtgtcc   6835
   cttcccagat tccctgacgt aagctgacat ctctcccag g cta ccc aag gaa gcc     6890
                                                Leu Pro Lys Glu Ala
                                                                510
   tgc atg gac atc tca tcg cca ggg tgt cag ctg cct gcc cag cgg ctg      6938
   Cys Met Asp Ile Ser Ser Pro Gly Cys Gln Leu Pro Ala Gln Arg Leu
                   515                 520                 525
   tgt cag cct gtg ccc ccc agc cca gcc tgc cag ctg gtt ttg cac cag      6986
   Cys Gln Pro Val Pro Pro Ser Pro Ala Cys Gln Leu Val Leu His Gln
               530                 535                 540
   gta ctg aag ggt ggc tca ggg acc tac tgc ctc aat gtg tct ttg gct      7034
   Val Leu Lys Gly Gly Ser Gly Thr Tyr Cys Leu Asn Val Ser Leu Ala
           545                 550                 555
   gat gcc aat agc ctg gcg atg gtc agc acc cag ctt gtc atg cct g        7080
   Asp Ala Asn Ser Leu Ala Met Val Ser Thr Gln Leu Val Met Pro
       560                 565                 570
   gtaggtagtt ggacaagagg taggatgaag acacggggag atggtagagg ttacctacta   7140
   gaggaagcag acactgaatg cagccgtatc tgggattcca cccatag gg  caa gaa      7195
                                                       Gly Gln Glu
                                                           575
   gca ggc ctc agg cag gct cct ctg ttc gtg ggc atc ttg ctg gtg cta      7243
   Ala Gly Leu Arg Gln Ala Pro Leu Phe Val Gly Ile Leu Leu Val Leu
               580                 585                 590
   aca gct ttg ttg ctt gca tct ctg ata tac ag  gtgagatccc cgccatcctg   7295
   Thr Ala Leu Leu Leu Ala Ser Leu Ile Tyr Arg
           595                     600
   ctcccactcc tttacccctt attaccacca ccactcttcc tcatgggaag aagaaaccac   7355
   caaccccttt gggaaagtgt agagtccaag aaagagccca gacttggaag ttcaacaggt   7415
   ctaggctgca gtcttgctgg tgggaccctg gggaagtcca ttaacccttc tgagccactg   7475
   aaaagtagga aacataatac ctgtcctgtg gggctgtttt cagggctcta gacaatgtga   7535
   gtaaaacacc tggttctgaa acaaaagtgg aataaatgat gatctcaatg actgttgtta   7595
   tgaataatat caacagtgga gaagaactca gtgaactgag ttctccacct gccagaaagg   7655
   caaatcccta ggcctggagg gctgaggtcc tcaaagcagg gaagcctgta gggtgagagg   7715
   gaaatggtca gagcttacca taaacataag agaggataaa ccctgttggt gagaagagga   7775
   gggagccagg atcaagacca agtcaacctg ggttatggtt tagtcttttt tttttagaga   7835
   agcacaaaga ggttgccatt gaccaccact aaccagtatc cctgcttttc tcccaatatc   7895
   ag g cga aga ctt atg aag caa ggc tca gca gtc ccc ctt ccc cag ctg    7943
        Arg Arg Leu Met Lys Gln Gly Ser Ala Val Pro Leu Pro Gln Leu
            605                 610                 615
   cca cac ggt aga acc cag tgg cta cgt ctg ccc tgg gtc ttc cgc tct    7991
   Pro His Gly Arg Thr Gln Trp Leu Arg Leu Pro Trp Val Phe Arg Ser
       620                 625                 630
   tgc ccc att ggt gag agc aaa ccc ctc ctc agt gga cag cag gtc        8036
   Cys Pro Ile Gly Glu Ser Lys Pro Leu Leu Ser Gly Gln Gln Val
   635                 640                 645
   tgagtgctct tatgtgaagt catgatttac ccaggtggac agcaaggcct gtcttttctc   8096
   tggtcttccc tcagagacta ccattgcctg aaataaagac tcagaacttg              8146
<210> 4
   <211> 649
   <212> PRT
   <213> Bos taurus
<400> 4
   Met Asp Leu Val Leu Arg Lys Tyr Leu Leu His Val Ala Leu Met Gly
   1 5 10 15
   Val Leu Leu Ala Val Arg Thr Thr Glu Gly Pro Arg Asp Arg Asp Trp
   20 25 30
   Leu Gly Val Ser Arg Gln Leu Arg Ile Lys Ala Trp Asn Arg Gln Leu
   35 40 45
   Tyr Pro Glu Trp Thr Glu Ser Gln Gly Pro Asp Cys Trp Arg Gly Gly
   50 55 60
   His Ile Ser Leu Lys Val Ser Asn Asp Gly Pro Thr Leu Ile Gly Ala
   65 70 75 80
   Asn Ala Ser Phe Ser Ile Ala Leu His Phe Pro Lys Ser Gln Lys Val
   85 90 95
   Leu Pro Asp Gly Gln Val Ile Trp Ala Asn Asn Thr Ile Ile Asn Gly
   100 105 110
   Ser Gln Val Trp Gly Gly Gln Leu Val Tyr Pro Gln Glu Pro Asp Asp
   115 120 125
   Thr Cys Ile Phe Pro Asp Gly Glu Pro Cys Pro Ser Gly Pro Leu Ser
   130 135 140
   Gln Lys Arg Cys Phe Val Tyr Val Trp Lys Thr Trp Asp Gln Tyr Trp
   145 150 155 160
   Gln Val Leu Gly Gly Pro Val Ser Gly Leu Ser Ile Gly Thr Asp Lys
   165 170 175
   Ala Met Leu Gly Thr Tyr Asn Met Glu Val Thr Val Tyr His Arg Arg
   180 185 190
   Gly Ser Gln Ser Tyr Val Pro Leu Ala His Ser Ser Ser Ala Phe Thr
   195 200 205
   Ile Thr Asp Gln Val Pro Phe Ser Val Ser Val Ser Gln Leu Gln Ala
   210 215 220
   Leu Asp Gly Arg Asn Lys Arg Phe Leu Arg Lys Gln Pro Leu Thr Phe
   225 230 235 240
   Ala Leu Gln Leu His Asp Pro Ser Gly Tyr Leu Ala Gly Ala Asp Leu
   245 250 255
   Ser Tyr Thr Trp Asp Phe Gly Asp Ser Thr Gly Thr Leu Ile Ser Arg
   260 265 270
   Ala Leu Thr Val Thr His Thr Tyr Leu Glu Ser Gly Pro Val Thr Ala
   275 280 285
   Gln Val Val Leu Gln Ala Ala Ile Pro Leu Thr Ser Cys Gly Ser Ser
   290 295 300
   Pro Val Pro Gly Thr Thr Asp Arg His Val Thr Thr Ala Glu Ala Pro
   305 310 315 320
   Gly Thr Thr Ala Gly Gln Val Pro Thr Thr Glu Val Met Gly Thr Thr
   325 330 335
   Pro Gly Gln Val Pro Thr Ala Glu Ala Pro Gly Thr Thr Val Gly Trp
   340 345 350
   Val Pro Thr Thr Glu Asp Val Gly Thr Thr Pro Glu Gln Val Ala Thr
   355 360 365
   Ser Lys Val Leu Ser Thr Thr Pro Val Glu Met Pro Thr Ala Lys Ala
   370 375 380
   Thr Gly Arg Thr Pro Glu Val Ser Thr Thr Glu Pro Ser Gly Thr Thr
   385 390 395 400
   Val Thr Gln Gly Thr Thr Pro Glu Leu Val Glu Thr Thr Ala Gly Glu
   405 410 415
   Val Ser Thr Pro Glu Pro Ala Gly Ser Asn Thr Ser Ser Phe Met Pro
   420 425 430
   Thr Glu Gly Thr Ala Gly Ser Leu Ser Pro Leu Pro Asp Asp Thr Ala
   435 440 445
   Thr Leu Val Leu Glu Lys Arg Gln Ala Pro Leu Asp Cys Val Leu Tyr
   450 455 460
   Arg Tyr Gly Ser Phe Ser Leu Thr Leu Asp Ile Val Gln Gly Ile Glu
   465 470 475 480
   Ser Ala Glu Ile Leu Gln Ala Val Ser Ser Ser Glu Gly Asp Ala Phe
   485 490 495
   Glu Leu Thr Val Ser Cys Gln Gly Gly Leu Pro Lys Glu Ala Cys Met
   500 505 510
   Asp Ile Ser Ser Pro Gly Cys Gln Leu Pro Ala Gln Arg Leu Cys Gln
   515 520 525
   Pro Val Pro Pro Ser Pro Ala Cys Gln Leu Val Leu His Gln Val Leu
   530 535 540
   Lys Gly Gly Ser Gly Thr Tyr Cys Leu Asn Val Ser Leu Ala Asp Ala
   545 550 555 560
   Asn Ser Leu Ala Met Val Ser Thr Gln Leu Val Met Pro Gly Gln Glu
   565 570 575
   Ala Gly Leu Arg Gln Ala Pro Leu Phe Val Gly Ile Leu Leu Val Leu
   580 585 590
   Thr Ala Leu Leu Leu Ala Ser Leu Ile Tyr Arg Arg Arg Leu Met Lys
   595 600 605
   Gln Gly Ser Ala Val Pro Leu Pro Gln Leu Pro His Gly Arg Thr Gln
   610 615 620
   Trp Leu Arg Leu Pro Trp Val Phe Arg Ser Cys Pro Ile Gly Glu Ser
   625 630 635 640
   Lys Pro Leu Leu Ser Gly Gln Gln Val
   645
<210> 5
   <211> 8143
   <212> ADN
   <213> Bos taurus
<220>
   <221> CDS
   <222> (30)..(102)
   <223>
<220>
   <221> CDS
<222> (2323)..(2433)
   <223>
<220>
   <221> CDS
   <222> (2579)..(2725)
   <223>
<220>
   <221> CDS
   <222> (3801)..(3935)
   <223>
<220>
   <221> CDS
   <222> (4312)..(4473)
   <223>
<220>
   <221> CDS
   <222> (4730)..(5410)
   <223>
<220>
   <221> CDS
   <222> (6318)..(6434)
   <223>
<220>
   <221> CDS
   <222> (6678)..(6762)
   <223>
<220>
   <221> CDS
   <222> (6872)..(7077)
   <223>
<220>
   <221> CDS
   <222> (7185)..(7272)
   <223>
<220>
   <221> CDS
   <222> (7895)..(8033)
   <223>
<400> 5
   ggtctttggt tgctggaagg aagaacagg atg gat ctg gtg ctg aga aaa tac       53
                                   Met Asp Leu Val Leu Arg Lys Tyr
                                   1               5
   ctt ctc cat gtg gct ctg atg ggt gtt ctg gct gta ggg acc aca gaa g   102
   Leu Leu His Val Ala Leu Met Gly Val Leu Ala Val Gly Thr Thr Glu
       10                  15                  20
   gtgagtgtgg gatgttggac atgaacaagt gtgaatttgg ggttgcacac ctgctctggt   162
   ttttctctcc ctaaaatgga agatatcagt agtgcttcag gtgtctccca cccatttgat   222
   ttagtgagga catgggcaac tgagctccct ccccacatga agatttgggt gcatgtgtgt   282
   tcaggcactt gggactgaac ctgaaaacaa ccccatctac ctggatgggt gagagaacag   342
   tatgtctccg tggccctaat tttgagatgc tctgaatagt gagctggaac atgggtgcca   402
   aggtagtaaa atgagtggaa actcatttag gctttgtctc aggcacttgg gatagggtat   462
   ttaggagata gagaaagata ggagatagga gaaaggagaa agaggatgtg gtattggata   522
   gaagggtaat gaggcacctc atcccctctt tgggatgggc atgggtgaac acagcccagg   582
   cttttgttct ggggctggaa gagacaggca gaagggtctc agctgagcat cacatgaaag   642
   ggctctgggg gattggggcc tcgtgacagg agcaaggcgg gtggggtggg gatggtgaga   702
   gggtctggaa tgtcccgtgc tgctctgagg agggaggatt gggagtggag aaagaatggg   762
   gcatcttatg attctcttgt tcttgtggtg aggtattcag tgggataatt ctagatcctc   822
   ccccaagaga atcaaccagg tttctggtac atgttagaga tggagtgagg atagtctgtg    882
   atgtgcagaa atatctacat tgtaccccag tgcccccttt ctctagatcc ctggtctcac    942
   agacttcttg gaacttctcc ttgatctgac ttccctcatt catggtgtca tttcaagtct   1002
   tattctttta ctatgttcgt tattgtattc tggaaatatc ctgttcatat gtgtccaccc   1062
   aaggctctta atatgttgtg cttacttttt ggatccagat ttttaaaatc ataagaagac   1122
   atttttatat agttcatgaa attttgcatg gactgagttt gataattttg tttagtgtga   1182
   attaacattg tgtttattta agaaaaaaaa atattttttt acagaaacct actgaatttg   1242
   tagggtttta aaataacatg atgtctggga tttgcttttg aatgcttcag ccaaaaaaca   1302
   aacgaacaac aaaaataaag gatagataaa gcaaatgtga caaaatgctg atagttgttg   1362
   gaccttgggg agacacatgc agagccatca catcactttt tttcagacat ctttcttggt   1422
   cagttataat cattttgttt gtccccactc ccaatttcta cttgcctcta gtccatcctc   1482
   ctcactgctt gccaaagtga tccttctaaa acacaaatct gatcatattc aaaaagcttt   1542
   tgaagggtaa gttttatggt atatgccata tatcagtaca acaaacaaaa tcgtctgagg   1602
   tgccgttgcc tacaggataa agtccaaact cctttgcctg gcactccaag cccccactct   1662
   atcttcttgg cctcatctct catgatgtac atcagccaca ttgctagtgt ctgctcatgg   1722
   ccttctgcct agaatgcttt atgccccagc caactattta ctgtcttctt cagtcgacca   1782
   gagtgcaatt tacctgttta aaatctatca ttttgttata cattgtgcat gtctattatg   1842
   gctcatatta agcaatgcct tggattatag taatttatgt atatgtctat ttcatatact   1902
   ttaacctgaa ccccttcaga accatttctt tttcatttct taagttcttt gcacctagcc   1962
   cagtgcctgg tacgtcgtgg gtattcagta gattaaaatg cactttaagg aacttccctt   2022
   gttgtccatc aagtggctaa ggctctgtgc tcccaatgca ggggaccagg gttcaatctc   2082
   aggtcaggga actagatccc acaggtcaca actaagagtt tgcaagccac aactacctga   2142
   cctcacatgc cacaactaat cgaagatccc tcgtgctgca actaagtcct agtgcagtta   2202
   aatatatttt tttaatgcac tttgaatgtg agaatgaatg atgtgtcaca gacactgttg   2262
   tcccctgaga agggagtgag taatgatttg agggccctca tagtatatct tcctttttag   2322
   ga  ccc aga gac agg gac tgg ctt ggt gtc tca agg cag ctc aga att     2369
   Gly Pro Arg Asp Arg Asp Trp Leu Gly Val Ser Arg Gln Leu Arg Ile
                       30                  35                  40
   aaa gca tgg aac aga cag ctg tat cca gag tgg aca gaa agc cag ggg     2417
   Lys Ala Trp Asn Arg Gln Leu Tyr Pro Glu Trp Thr Glu Ser Gln Gly
                   45                  50                  55
   cct gac tgc tgg aga g gtaggaactt ggcaatttcc agggaggata tggtggaaat   2473
   Pro Asp Cys Trp Arg
               60
   gggtggggag gggaacgggg ttgaatgtac ttaggaagat agggaaggaa aaggcataca   2533
   gggaggagaa gccaaggagc taattaatgc agctgccctt ttcag gt  ggc cac ata   2589
                                                     Gly Gly His Ile
                                                                 65
   tcc ctg aag gtc agc aat gat ggg cct aca ctg att ggg gca aat gct     2637
   Ser Leu Lys Val Ser Asn Asp Gly Pro Thr Leu Ile Gly Ala Asn Ala
                   70                  75                  80
   tcc ttc tct att gcc ttg cac ttt cct aaa agc caa aag gtg ctg cca     2685
   Ser Phe Ser Ile Ala Leu His Phe Pro Lys Ser Gln Lys Val Leu Pro
               85                  90                  95
   gat ggg cag gtc atc tgg gcc aac aac acc atc atc aat g gtgagtacct    2735
   Asp Gly Gln Val Ile Trp Ala Asn Asn Thr Ile Ile Asn
           100                 105                 110
   ctccgcctcc ttcccaaggt ccagaatccc tggtatcccc aatgagctca aggaatcctc   2795
   ctcctctttt tttttttttt tttttacaaa ttatatatgt aacacatatt cactgcagaa   2855
   aaattagaaa acacagataa accaaaaaga aaaaaaatta tagttcccca aatggggcac   2915
   agaagaccca gtggacatag aagttggata gacttggatt taaactggtt accagtatgt   2975
   gaccctggac aagtcactga attgttttgt tcttccattc ccttatctat agaatgggga   3035
   tgataacact ttaaaaggtt cttgtaagga ttaaaatgtg ataatatata aagattttag   3095
   cataatgcct gccctgtgct gtgcttagta ccttagttta gacgctttgc aaccccatgg   3155
   actgtagccc accaggctcc tctgtccatg tggattctgc aggcaagaat actggagtgg   3215
   gtcaccatgc actcctccag gggatcttcc caactcaggg atcgaaccca ggtcctagcc   3275
   tacagtatta attgatgctg ttatttttac ttttatccca ctagctagag cacatcatcc   3335
   tagacatttt gatacatggc ctaccaattt gtgtccagtg taagaatata catgtgtgtg   3395
   ctcagtggct cagtcgtgtc tgactctttg caaccccatg gactgtagcc cgcgaaagct   3455
   cctctgccca tgggattgcc cagccaagaa tactggagca ggttgccatt tcttcctcca   3515
   ggggatcttt caacacaggg attgaatcct tgtctcctgt gtttcctgca ttggcaggtg   3575
   tattctttac cactgagcca cctgggaaac cccttaagta tatacacata aatcttttat   3635
   agtttccatt ctcccttcta ccactccaaa taggttatac caaggagaat gtattttggt   3695
   agctaggcag tattcctgga gcccctctct gggagtcatg ttaaaggttt tggtgtacag   3755
   tgaggaatgc cagggattga gggagacttg ctgtcttctt ttcag gg agc cag gtg   3811
                                                   Gly Ser Gln Val
   tgg gga gga cag ctg gta tat ccc caa gaa cct gat gat acc tgc atc   3859
   Trp Gly Gly Gln Leu Val Tyr Pro Gln Glu Pro Asp Asp Thr Cys Ile
   115                 120                 125                 130
   ttc ccc gat ggg gag ccc tgc cct tct ggc cct cta tct cag aaa aga   3907
   Phe Pro Asp Gly Glu Pro Cys Pro Ser Gly Pro Leu Ser Gln Lys Arg
                   135                 140                 145
   tgc ttt gtt tat gtc tgg aag acc tgg g gtaagagttt cccttctctg       3955
   Cys Phe Val Tyr Val Trp Lys Thr Trp
               150                 155
   gcctgtcatt cacacttaaa ttcacttctt cctacctgat cccctttctt ttggtctcat   4015
   ccttaaattc tgtgagtttc cctaatcttc acttccccca tgactccttc ctcttccaca   4075
   gcacctagtc aactctatta tacttctttc tgggagccct gctccaatta tagtcccatc   4135
   ccatggaccc tctcataagg acttttttcc tgcccaacat atgcaagctt aaactctctg   4195
   aaataaccat ccttgataca tctcctgacc ttccttctct ggttccatct ctaaccctgc   4255
   cccagtctcc tttgaccagt aacccccttc cctactcttc tttccaaaaa cctcag ac    4313
                                                                 Asp
   caa tac tgg caa gtt ctg ggg ggc cca gtg tct gga ctg agc atc ggg   4361
   Gln Tyr Trp Gln Val Leu Gly Gly Pro Val Ser Gly Leu Ser Ile Gly
               160                 165                 170
   aca gac aag gca atg ctg ggc aca tat aac atg gaa gtg act gtc tac   4409
   Thr Asp Lys Ala Met Leu Gly Thr Tyr Asn Met Glu Val Thr Val Tyr
           175                 180                 185
   cac cgc cgg ggg tcc cag agc tat gtg ccc ctc gct cac tcc agt tca   4457
   His Arg Arg Gly Ser Gln Ser Tyr Val Pro Leu Ala His Ser Ser Ser
       190                 195                 200
   gcc ttc acc att act g gtaaggactg aggaggggac aaggccagtt gcagggcagg   4513
   Ala Phe Thr Ile Thr
   205
   agaaggtggg gaggctgggc tggacaggaa aggggaaaga ggaaatggtg tgtaacctta   4573
   caggggcaga accaggaaga tgtgggcaga gggatgtggg gcttggagcc cgtgaagggc   4633
   caggcagctt gggttggttg aaaaatatgg ctgtgaaaga agaagctgac agaaagaaga   4693
   acttatggtt ctcactttct ctgactccaa tcccag ac  cag gtg ccc ttc tct     4746
                                            Asp Gln Val Pro Phe Ser
                                                                215
   gtg agt gtg tct cag ctg cag gcc ttg gat gga agg aac aag cgc ttc     4794
   Val Ser Val Ser Gln Leu Gln Ala Leu Asp Gly Arg Asn Lys Arg Phe
                   220                 225                 230
   ctg aga aag cag cct ctg acc ttt gcc ctc cag ctc cat gat ccc agt     4842
   Leu Arg Lys Gln Pro Leu Thr Phe Ala Leu Gln Leu His Asp Pro Ser
               235                 240                 245
   ggc tat ttg gct ggg gct gac ctt tcc tac acc tgg gac ttt ggt gac     4890
   Gly Tyr Leu Ala Gly Ala Asp Leu Ser Tyr Thr Trp Asp Phe Gly Asp
           250                 255                 260
   agt aca ggg acc ctg atc tct cgg gca ctc acg gtc act cac act tac     4938
   Ser Thr Gly Thr Leu Ile Ser Arg Ala Leu Thr Val Thr His Thr Tyr
       265                 270                 275
   cta gag tct ggc cca gtc act gca cag gtg gtg ctg cag gct gcc att     4986
   Leu Glu Ser Gly Pro Val Thr Ala Gln Val Val Leu Gln Ala Ala Ile
   280                 285                 290                 295
   cct ctc acc tcc tgt ggc tcc tct cca gtt cca ggc act aca gat agg     5034
   Pro Leu Thr Ser Cys Gly Ser Ser Pro Val Pro Gly Thr Thr Asp Arg
                   300                 305                 310
   cat gtg aca act gca gag gct cct gga acc aca gct ggc caa gtg cct     5082
   His Val Thr Thr Ala Glu Ala Pro Gly Thr Thr Ala Gly Gln Val Pro
               315                 320                 325
   act aca gaa gtc atg ggc acc aca cct ggc cag gtg cca act gca gag     5130
   Thr Thr Glu Val Met Gly Thr Thr Pro Gly Gln Val Pro Thr Ala Glu
           330                 335                 340
   gcc cct ggc acc aca gtt ggg tgg gtg cca acc aca gag gat gta ggt     5178
   Ala Pro Gly Thr Thr Val Gly Trp Val Pro Thr Thr Glu Asp Val Gly
       345                 350                 355
   acc aca cct gag cag gtg gca acc tcc aaa gtc tta agt aca aca cca     5226
   Thr Thr Pro Glu Gln Val Ala Thr Ser Lys Val Leu Ser Thr Thr Pro
   360                 365                 370                 375
   gtg gag atg cca act gca aaa gct aca ggt agg aca cct gaa gtg tca     5274
   Val Glu Met Pro Thr Ala Lys Ala Thr Gly Arg Thr Pro Glu Val Ser
                   380                 385                 390
   act aca gag ccc tct gga acc aca gtt aca cag gga aca act cca gag     5322
   Thr Thr Glu Pro Ser Gly Thr Thr Val Thr Gln Gly Thr Thr Pro Glu
               395                 400                 405
   ctg gtg gag acc aca gct gga gag gtg tcc act cct gag cct gcg ggt     5370
   Leu Val Glu Thr Thr Ala Gly Glu Val Ser Thr Pro Glu Pro Ala Gly
           410                 415                 420
   tca aat act agc tca ttc atg cct aca gaa ggt act gca g gtaagggggc     5420
   Ser Asn Thr Ser Ser Phe Met Pro Thr Glu Gly Thr Ala
       425                 430                 435
   caccatgaat gagttcatag aggtggggca tttgtcacag ctctgaagac ctgaaagaat   5480
   tgctcaggac ccagatgtta ctcaatcctt agcttagcag tggagtcccc tcagaatctt   5540
   cactggtttt aaaaccccct aagtccctct taatggcaca gaatagatcc agagttcagg   5600
   aaaccagggt cttctcctag gccaggggta gagagcttat tctctcttcc tgaagagaag   5660
   ttcaggaagc agtgtgtgat catttggtgg tggtgctcag tcatgtctga ctctttgtga   5720
   cctcatggac tatggcccac caggctcctc tgtccataga attctccagg caagaacact   5780
   ggagtgggtg gccatttcct tctccagggg attttccctg cccagggatt aaacccgaat   5840
   tggcaggtgg attctttacc cgagccacct agaaagtccc atgtgatcat tagataatac   5900
   ttatacctca ttttctgatt aagtgtaaac acagaaatct ttctgacacc acttcccacc   5960
   cctggattcc catcccaaag taggtttacc tggaattgtg gtaggaatac taaaaaggga   6020
   gaagtgagat agtgacacta tgacttaaca catgtcaaat gtctgaccca ggacctggca   6080
   cagtgtaggg tgtgataaac atttgggatg tctaaaattc tgactctaac cctgtgactc   6140
   tggggcagtc atttctcttg ggcctttctt tatcttaaaa aatgagagtt tccagctctt   6200
   gtctgattct aagcctggat ccagtagctc tgactctacc tggaaaaatg cttgttgggc   6260
   ctgttttcag gttagtcatt tgctttttga ctttgcctct ttaatcctct cctccag      6317
   gc  tcc ctg agt ccc ctg ccg gat gac act gcc acc tta gtc ctg gag     6364
   Gly Ser Leu Ser Pro Leu Pro Asp Asp Thr Ala Thr Leu Val Leu Glu
               440                 445                 450
   aag cgc caa gcc ccc ctg gat tgt gtt ctg tat cgc tat ggc tcc ttt     6412
   Lys Arg Gln Ala Pro Leu Asp Cys Val Leu Tyr Arg Tyr Gly Ser Phe
           455                 460                 465
   tcc ctc acc ctg gac att gtc c gtgagtcttg cctacattgt ccgtaagctg      6464
   Ser Leu Thr Leu Asp Ile Val
       470                 475
   gtggagggag gcgtgtgctg cttagggttg cccagtggaa gcacaccttg gaaggaatta   6524
   ctcacctgga caaggagaat acccagatcc caggggtttc atatgaaggc agaatgggat   6584
   tagggaggca gcccgaggac cttcctggcc atgggccttg ggggaggata agtagaggag   6644
   tctcagactt aaaaaaatct tgcaactttg cag ag  ggt att gag agt gct gag    6697
                                           Gln Gly Ile Glu Ser Ala Glu
                                                               480
   atc cta cag gct gtg tca tcc agt gaa gga gat gca ttt gag ctg act     6745
   Ile Leu Gln Ala Val Ser Ser Ser Glu Gly Asp Ala Phe Glu Leu Thr
           485                 490                 495
   gtg tct tgc caa ggc gg  gtgagtgtcc cacggttgcc ctgagaactc            6792
   Val Ser Cys Gln Gly Gly
       500
   ctggggtgac tgctgtcctg ttctctggtg tctagtgtcc cttcccagat tccctgacgt   6852
   aagctgacat ctctcccag g cta ccc aag gaa gcc tgc atg gac atc tca      6902
                          Leu Pro Lys Glu Ala Cys Met Asp Ile Ser
                          505                 510
   tcg cca ggg tgt cag ctg cct gcc cag cgg ctg tgt cag cct gtg ccc     6950
   Ser Pro Gly Cys Gln Leu Pro Ala Gln Arg Leu Cys Gln Pro Val Pro
   515                 520                 525                 530
   ccc agc cca gcc tgc cag ctg gtt ttg cac cag gta ctg aag ggt ggc     6998
   Pro Ser Pro Ala Cys Gln Leu Val Leu His Gln Val Leu Lys Gly Gly
                   535                 540                 545
   tca ggg acc tac tgc ctc aat gtg tct ttg gct gat gcc aat agc ctg     7046
   Ser Gly Thr Tyr Cys Leu Asn Val Ser Leu Ala Asp Ala Asn Ser Leu
               550                 555                 560
   gcg atg gtc agc acc cag ctt gtc atg cct g gtaggtagtt ggacaagagg     7097
   Ala Met Val Ser Thr Gln Leu Val Met Pro
           565                 570
   taggatgaag acacggggag atggtagagg ttacctacta gaggaagcag acactgaatg   7157
   cagccgtatc tgggattcca cccatag gg  caa gaa gca ggc ctc agg cag gct   7210
                                     Gly Gln Glu Ala Gly Leu Arg Gln Ala
                                             575                 580
   cct ctg ttc gtg ggc atc ttg ctg gtg cta aca gct ttg ttg ctt gca     7258
   Pro Leu Phe Val Gly Ile Leu Leu Val Leu Thr Ala Leu Leu Leu Ala
               585                 590                 595
   tct ctg ata tac ag  gtgagatccc cgccatcctg ctcccactcc tttacccctt     7312
   Ser Leu Ile Tyr Arg
           600
   attaccacca ccactcttcc tcatgggaag aagaaaccac caaccccttt gggaaagtgt   7372
   agagtccaag aaagagccca gacttggaag ttcaacaggt ctaggctgca gtcttgctgg   7432
   tgggaccctg gggaagtcca ttaacccttc tgagccactg aaaagtagga aacataatac   7492
   ctgtcctgtg gggctgtttt cagggctcta gacaatgtga gtaaaacacc tggttctgaa   7552
   acaaaagtgg aataaatgat gatctcaatg actgttgtta tgaataatat caacagtgga   7612
   gaagaactca gtgaactgag ttctccacct gccagaaagg caaatcccta ggcctggagg   7672
   gctgaggtcc tcaaagcagg gaagcctgta gggtgagagg gaaatggtca gagcttacca   7732
   taaacataag agaggataaa ccctgttggt gagaagagga gggagccagg atcaagacca   7792
   agtcaacctg ggttatggtt tagtcttttt tttttagaga agcacaaaga ggttgccatt   7852
   gaccaccact aaccagtatc cctgcttttc tcccaatatc ag g cga aga ctt atg    7907
                                                    Arg Arg Leu Met
                                                            605
   aag caa ggc tca gca gtc ccc ctt ccc cag ctg cca cac ggt aga acc     7955
   Lys Gln Gly Ser Ala Val Pro Leu Pro Gln Leu Pro His Gly Arg Thr
               610                 615                 620
   cag tgg cta cgt ctg ccc tgg gtc ttc cgc tct tgc ccc att ggt gag     8003
   Gln Trp Leu Arg Leu Pro Trp Val Phe Arg Ser Cys Pro Ile Gly Glu
           625                 630                 635
   agc aaa ccc ctc ctc agt gga cag cag gtc tgagtgctct tatgtgaagt       8053
   Ser Lys Pro Leu Leu Ser Gly Gln Gln Val
       640                 645
   catgatttac ccaggtggac agcaaggcct gtcttttctc tggtcttccc tcagagacta   8113
   ccattgcctg aaataaagac tcagaacttg                                    8143
<210> 6
   <211> 648
   <212> PRT
   <213> Bos taurus
<400> 6
   Met Asp Leu Val Leu Arg Lys Tyr Leu Leu His Val Ala Leu Met Gly
   1               5                   10                  15
   Val Leu Ala Val Gly Thr Thr Glu Gly Pro Arg Asp Arg Asp Trp Leu
               20                  25                  30
   Gly Val Ser Arg Gln Leu Arg Ile Lys Ala Trp Asn Arg Gln Leu Tyr
           35                  40                  45
   Pro Glu Trp Thr Glu Ser Gln Gly Pro Asp Cys Trp Arg Gly Gly His
       50                  55                  60
   Ile Ser Leu Lys Val Ser Asn Asp Gly Pro Thr Leu Ile Gly Ala Asn
   65                  70                  75                  80
   Ala Ser Phe Ser Ile Ala Leu His Phe Pro Lys Ser Gln Lys Val Leu
                   85                  90                  95
   Pro Asp Gly Gln Val Ile Trp Ala Asn Asn Thr Ile Ile Asn Gly Ser
               100                 105                 110
   Gln Val Trp Gly Gly Gln Leu Val Tyr Pro Gln Glu Pro Asp Asp Thr
           115                 120                 125
   Cys Ile Phe Pro Asp Gly Glu Pro Cys Pro Ser Gly Pro Leu Ser Gln
       130                 135                 140
   Lys Arg Cys Phe Val Tyr Val Trp Lys Thr Trp Asp Gln Tyr Trp Gln
   145                 150                 155                 160
   Val Leu Gly Gly Pro Val Ser Gly Leu Ser Ile Gly Thr Asp Lys Ala
                   165                 170                 175
   Met Leu Gly Thr Tyr Asn Met Glu Val Thr Val Tyr His Arg Arg Gly
               180                 185                 190
   Ser Gln Ser Tyr Val Pro Leu Ala His Ser Ser Ser Ala Phe Thr Ile
           195                 200                 205
   Thr Asp Gln Val Pro Phe Ser Val Ser Val Ser Gln Leu Gln Ala Leu
       210                 215                 220
   Asp Gly Arg Asn Lys Arg Phe Leu Arg Lys Gln Pro Leu Thr Phe Ala
   225                 230                 235                 240
   Leu Gln Leu His Asp Pro Ser Gly Tyr Leu Ala Gly Ala Asp Leu Ser
                   245                 250                 255
   Tyr Thr Trp Asp Phe Gly Asp Ser Thr Gly Thr Leu Ile Ser Arg Ala
               260                 265                 270
   Leu Thr Val Thr His Thr Tyr Leu Glu Ser Gly Pro Val Thr Ala Gln
           275                 280                 285
   Val Val Leu Gln Ala Ala Ile Pro Leu Thr Ser Cys Gly Ser Ser Pro
       290                 295                 300
   Val Pro Gly Thr Thr Asp Arg His Val Thr Thr Ala Glu Ala Pro Gly
   305                 310                 315                 320
   Thr Thr Ala Gly Gln Val Pro Thr Thr Glu Val Met Gly Thr Thr Pro
                   325                 330                 335
   Gly Gln Val Pro Thr Ala Glu Ala Pro Gly Thr Thr Val Gly Trp Val
               340                 345                 350
   Pro Thr Thr Glu Asp Val Gly Thr Thr Pro Glu Gln Val Ala Thr Ser
   355 360 365
   Lys Val Leu Ser Thr Thr Pro Val Glu Met Pro Thr Ala Lys Ala Thr
   370 375 380
   Gly Arg Thr Pro Glu Val Ser Thr Thr Glu Pro Ser Gly Thr Thr Val
   385 390 395 400
   Thr Gln Gly Thr Thr Pro Glu Leu Val Glu Thr Thr Ala Gly Glu Val
   405 410 415
   Ser Thr Pro Glu Pro Ala Gly Ser Asn Thr Ser Ser Phe Met Pro Thr
   420 425 430
   Glu Gly Thr Ala Gly Ser Leu Ser Pro Leu Pro Asp Asp Thr Ala Thr
   435 440 445
   Leu Val Leu Glu Lys Arg Gln Ala Pro Leu Asp Cys Val Leu Tyr Arg
   450 455 460
   Tyr Gly Ser Phe Ser Leu Thr Leu Asp Ile Val Gln Gly Ile Glu Ser
   465 470 475 480
   Ala Glu Ile Leu Gln Ala Val Ser Ser Ser Glu Gly Asp Ala Phe Glu
   485 490 495
   Leu Thr Val Ser Cys Gln Gly Gly Leu Pro Lys Glu Ala Cys Met Asp
   500 505 510
   Ile Ser Ser Pro Gly Cys Gln Leu Pro Ala Gln Arg Leu Cys Gln Pro
   515 520 525
   Val Pro Pro Ser Pro Ala Cys Gln Leu Val Leu His Gln Val Leu Lys
   530 535 540
   Gly Gly Ser Gly Thr Tyr Cys Leu Asn Val Ser Leu Ala Asp Ala Asn
   545 550 555 560
   Ser Leu Ala Met Val Ser Thr Gln Leu Val Met Pro Gly Gln Glu Ala
   565 570 575
   Gly Leu Arg Gln Ala Pro Leu Phe Val Gly Ile Leu Leu Val Leu Thr
   580 585 590
   Ala Leu Leu Leu Ala Ser Leu Ile Tyr Arg Arg Arg Leu Met Lys Gln
   595 600 605
   Gly Ser Ala Val Pro Leu Pro Gln Leu Pro His Gly Arg Thr Gln Trp
   610 615 620
   Leu Arg Leu Pro Trp Val Phe Arg Ser Cys Pro Ile Gly Glu Ser Lys
   625 630 635 640
   Pro Leu Leu Ser Gly Gln Gln Val
   645
<210> 7
   <211> 294
   <212> ADN
   <213> Séquence artificielle
<220>
   <223> Sonde
<400> 7
   gttgctggaa ggaagaacag gatggatctg gtgctgagaa aataccttct ccatgtggct     60
   ctgatgggtg ttcttctggc tgtagggacc acagaaggtg agtgtgggat gttggacatg    120
   aacaagtgtg aatttggggt tgcacacctg ctctggtttt tctctcccta aaatggaaga    180
   tatcagtagt gcttcaggtg tctcccaccc atttgattta gtgaggacat gggcaactga    240
   gctccctccc cacatgaaga tttgggtgca tgtgtgttca ggcacttggg actg          294
<210> 8
   <211> 294
   <212> ADN
   <213> Séquence artificielle
<220>
   <223> Sonde
<400> 8
   gttgctggaa ggaagaacag gatggatctg gtgctgagaa aataccttct ccatgtggct     60
   ctgatgggtg ttcttctggc tgtaaggacc acagaaggtg agtgtgggat gttggacatg    120
   aacaagtgtg aatttggggt tgcacacctg ctctggtttt tctctcccta aaatggaaga    180
   tatcagtagt gcttcaggtg tctcccaccc atttgattta gtgaggacat gggcaactga    240
   gctccctccc cacatgaaga tttgggtgca tgtgtgttca ggcacttggg actg          294
<210> 9
   <211> 291
   <212> ADN
   <213> Séquence artificielle
<220>
   <223> Sonde
<400> 9
   gttgctggaa ggaagaacag gatggatctg gtgctgagaa aataccttct ccatgtggct     60
   ctgatgggtg ttctggctgt agggaccaca gaaggtgagt gtgggatgtt ggacatgaac    120
   aagtgtgaat ttggggttgc acacctgctc tggtttttct ctccctaaaa tggaagatat    180
   cagtagtgct tcaggtgtct cccacccatt tgatttagtg aggacatggg caactgagct    240
   ccctccccac atgaagattt gggtgcatgt gtgttcaggc acttgggact g             291
<210> 10
   <211> 30
   <212> ADN
   <213> Séquence artificielle
<220>
   <223> Amorce
<400> 10
   gttgctggaa ggaagaacag gatggatctg 30
<210> 11
   <211> 30
   <212> ADN
   <213> Séquence artificielle
<220>
   <223> Amorce
<400> 11
   cagtcccaag tgcctgaaca cacatgcacc 30

## Description

L'invention a pour objet l'utilisation de séquences nucléotidiques correspondant au gène *SILVER,* encore désigné gène *SI,* et aux différentes formes allèliques de ce gène, ou correspondant à des fragments de ce gène ou de ses différentes formes allèliques, pour la mise en oeuvre d'un procédé d'identification des différentes populations ou races des mammifères ruminants tels que les bovins, les ovins, et les caprins.

Depuis les récentes crises alimentaires en Europe, le consommateur veut être informé sur l'origine du produit : la notion de race est devenue déterminante pour les filières bovines de qualité, car elle est associée à un système d'élevage valorisant le terroir.

Le cheptel bovin français compte plus de 20 millions de têtes réparties en une quarantaine de races qui ont généralement une origine régionale bien identifiée et une couleur de robe typique, héritée du travail des premiers éleveurs sélectionneurs aux 18^{ème} et 19^{ème} siècles. Dans le cas de la production de viande bovine de qualité, la race d'origine du produit est une composante déterminante du cahier des charges, que l'éleveur veut garantir au consommateur. Le nom de la race devient une marque mise en valeur par le distributeur (par exemple le label rouge Blason Prestige pour la race Limousine, le label rouge Boeuf Gascon, ou l'AOC Maine-Anjou avec la race Rouge des Prés). Ainsi, les étiquettes des produits, 'label,' précisent l'origine, le cahier des charges, le type d'alimentation et la race, mais présentent aussi le produit sur pied avec une photographie, ce qui montre la valeur informative de l'aspect des animaux, dont la couleur est une composante majeure. Par ailleurs, la coloration de la robe a joué un rôle déterminant dans l'histoire des races bovines : alors qu'une race bovine présente une variabilité des performances et du génome, elle est précisément décrite et fixée pour sa couleur, qui devient donc un élément caractéristique de l'appartenance raciale d'un animal, et des produits qui en dérivent.

Rouzaud et al. décrivent un essai de génotypage des bovines françaises basé sur la gène extension" " (Genet. Sel. Evol., 32, 511-20, 2000).

La présente invention découle de la mise en évidence par les Inventeurs d'un gène majeur de la coloration chez les bovins, à savoir du gène *SILVER,* et du fait que le polymorphisme de ce gène chez les animaux est une caractéristique de l'appartenance raciale d'un animal. Deux allèles de ce gène ont été identifiés. Le premier, l'allèle *si,* propre à la race charolaise est causal(e) de la coloration blanc crème de sa robe. Le deuxième, *si₁* affecte la même région que l'allèle charolais et caractérise une autre race bovine, à savoir la race simmental.

Si les marqueurs moléculaires anonymes comme les microsatellites permettent d'attribuer un animal à une race avec une probabilité élevée, ils ne permettent pas pour autant de garantir le respect de tous les critères définissant la race et s'avèrent très peu efficaces dans le cas d'échantillons provenant d'animaux croisés. En revanche, l'invention permet de garantir que les produits commercialisés respectent les critères attachés à la race, et en particulier son standard, attesté par la couleur de la robe. Par ailleurs, pour proposer un test moléculaire de traçabilité individuelle, il faudrait génotyper tous les animaux abattus, afin d'établir la correspondance entre le typage d'un échantillon de viande et le génotype d'un animal donné. Pour la traçabilité raciale, un seul typage est à réaliser pour être comparé au génotype de référence, caractéristique de la race. Le test de traçabilité raciale de la présente invention est plus simple à mettre en oeuvre qu'un test de traçabilité individuelle. Dans ce contexte, l'invention permet aujourd'hui de donner aux éleveurs sélectionneurs de la race charolaise un moyen nouveau de garantir la traçabilité raciale de leurs animaux et de permettre, par les mêmes moyens, à tout opérateur économique, de vérifier la fiabilité de son fournisseur dans le cadre de ses accords contractuels, indépendamment d'un dispositif officiel de certification de produits.

L'invention a pour objet l'utilisation de séquences nucléotidiques correspondant au gène *SILVER,* encore désigné gène *SI*, représenté par SEQ ID No : 1 et codant pour la protéine bovine représentée par SEQ ID No : 2, et aux différentes formes allèliques de ce gène, ou correspondant à des fragments de ce gène ou de ses différentes formes allèliques, pour la mise en oeuvre d'un procédé d'identification des différentes populations ou races des mammifères ruminants tels que les bovins, les ovins, et les caprins.

L'invention a plus particulièrement pour objet l'utilisation susmentionnée, de séquences nucléotidiques correspondant au gène *SILVER* bovin représenté par SEQ ID NO : 1, codant pour la protéine bovine SI représentée par SEQ ID NO : 2, et aux différentes formes allèliques de ce gène, ou correspondant à des fragments de ce gène ou de ses différentes formes allèliques, pour la mise en oeuvre d'un procédé d'identification des différentes populations ou races bovines, ou de différents troupeaux bovins regroupant chacun plusieurs populations ou races bovines.

L'invention concerne plus particulièrement l'utilisation de séquences nucléotidiques telles que définies ci-dessus, pour la mise en oeuvre d'une méthode permettant de contrôler, voire de certifier, l'appartenance d'un animal à une population ou race bovine particulière, ou à un troupeau bovin particulier, ou, au contraire, permettant de certifier l'exclusion de cet animal de cette population ou race, ou de ce troupeau particulier.

L'invention a plus particulièrement pour objet l'utilisation de séquences nucléotidiques telles que définies ci-dessus, caractérisée en ce que les populations ou races bovines ou troupeaux bovins sont d'origine française.

L'invention concerne également l'utilisation susmentionnée :
- de la séquence nucléotidique correspondant à la forme allèlique *si* représentée par SEQ ID NO :3, codant pour la protéine bovine si représentée par SEQ ID NO : 4, ou correspondant à des fragments de cette forme allèlique, ladite forme allèlique comprenant la mutation G93A par rapport au gène *SI*, pour la mise en oeuvre d'une méthode permettant de contrôler, voire de certifier, l'appartenance d'un animal à la race charolaise,
- de la séquence nucléotidique correspondant à la forme allèlique *si₁* représentée par SEQ ID NO : 5, codant pour la protéine bovine si₁ représentée par SEQ ID NO : 6, ou correspondant à des fragments de cette forme allèlique, ladite forme allèlique comprenant une délétion des trois nucléotides TTC situés aux positions 82, 83 et 84 par rapport au gène *SI,* pour la mise en oeuvre d'une méthode permettant de contrôler, voire de certifier, l'appartenance d'un animal à la race simmental,
- de la séquence nucléotidique correspondant au gène *SI* bovin représenté par SEQ ID NO : 1, ou à des fragments de ce gène, pour la mise en oeuvre d'une méthode permettant de certifier l'exclusion d'un animal de la race charolaise.

L'invention concerne également l'utilisation susmentionnée, de fragments des séquences nucléotidiques correspondant aux formes allèliques *SI, si,* et *si₁*, représentées respectivement par SEQ ID NO : 1, 3, et 5, lesdits fragments étant choisis parmi ceux d'environ 10 à 300 nucléotides contenant les nucléotides situés aux positions 82 à 93 desdites séquences.

L'invention concerne plus particulièrement l'utilisation susmentionnée, de fragments des séquences nucléotidiques correspondant aux formes allèliques *SI, si,* et *si₁*, lesdits fragments étant choisis parmi ceux de 294 nucléotides délimités par les nucléotides situés aux positions 9 et 302 des séquences SEQ ID NO : 1 et 3, ces fragments étant représentés respectivement par les séquences SEQ ID NO : 7, et SEQ ID NO : 8, et le fragment de 291 nucléotides délimité par les nucléotides situés aux positions 9 et 299 de la séquence SEQ ID NO : 5, ce fragment étant représenté par la séquence SEQ ID NO : 9.

L'invention a également pour objet l'utilisation d'amorces nucléotidiques permettant d'amplifier le nombre de copies du gène *SILVER,* ou des différentes formes allèliques de ce gène, ou des fragments de ce gène ou de ses différentes formes allèliques, tels que définis ci-dessus, pour la mise en oeuvre d'un procédé d'identification des différentes populations ou races des mammifères ruminants tels que les bovins, les ovins, et les caprins.

L'invention concerne plus particulièrement l'utilisation d'amorces nucléotidiques susmentionnées, sous forme de couples d'amorces 5'-3', ces couples étant tels que :
- l'amorce 5' est choisie parmi l'amorce SIL10 représentée par la séquence SEQ ID NO : 10 suivante :
   5' GTTGCTGGAAGGAAGAACAGGATGGATCTG 3'
   ou toute séquence dérivée de cette séquence SEQ ID NO : 10, notamment par suppression et/ou substitution et/ou addition d'un ou plusieurs nucléotides, ladite séquence dérivée s'hybridant, tout comme la séquence SEQ ID NO : 10, avec tout ou partie de la séquence nucléotidique complémentaire des séquences nucléotidiques délimitées par les nucléotides situés aux positions 9 et 38 des séquences SEQ ID NO : 1, 3, et 5,
- l'amorce 3' est choisie parmi l'amorce SIL8 représentée par la séquence SEQ ID NO : 11 suivante :
   5'CAGTCCCAAGTGCCTGAACACACATGCACC3'
   ou toute séquence dérivée de cette séquence SEQ ID NO : 11, notamment par suppression et/ou substitution et/ou addition d'un ou plusieurs nucléotides, ladite séquence dérivée s'hybridant, tout comme la séquence SEQ ID NO : 11, avec tout ou partie des séquences nucléotidiques délimitées par les nucléotides situés aux positions 276 et 302 des séquences SEQ ID NO : 1, 3, et par les nucléotides situés aux positions 273 et 299, de la séquence SEQ ID NO : 5.

L'invention concerne également la séquence nucléotidique caractérisée en ce qu'elle correspond au gène *SI* bovin représenté par SEQ ID NO : 1, ou aux fragments suivants du gène *SI :*
- tout fragment choisi parmi ceux d'environ 10 à 300 nucléotides contenant les nucléotides situés aux positions 82 à 93 de la séquence SEQ ID NO : 1,
- le fragment SEQ ID NO : 7 de 294 nucléotides délimité par les nucléotides situés aux positions 9 et 302 de la séquence SEQ ID NO : 1.

L'invention a également pour objet la séquence nucléotidique caractérisée en ce qu'elle correspond au gène *si* bovin représenté par SEQ ID NO : 3, ou aux fragments suivants du gène *si* :
- tout fragment choisi parmi ceux d'environ 10 à 300 nucléotides contenant les nucléotides situés aux positions 82 à 93 de la séquence SEQ ID NO : 3,
- le fragment SEQ ID NO : 8 de 294 nucléotides délimité par les nucléotides situés aux positions 9 et 302 de la séquence SEQ ID NO : 3.

L'invention concerne également la séquence nucléotidique caractérisée en ce qu'elle correspond au gène *si₁* bovin représenté par SEQ ID NO : 5, ou aux fragments suivants du gène *si₁* :
- tout fragment choisi parmi ceux d'environ 10 à 300 nucléotides contenant les nucléotides situés aux positions 82 à 93 de la séquence SEQ ID NO : 5,
- le fragment SEQ ID NO : 9 de 291 nucléotides délimité par les nucléotides situés aux positions 9 et 299 de la séquence SEQ ID NO : 5.

L'invention a également pour objet la séquence nucléotidique caractérisée en ce qu'elle comprend :
- la séquence nucléotidique SIL10 représentée par la séquence SEQ ID NO : 10 suivante :
   5' GTTGCTGGAAGGAAGAACAGGATGGATCTG 3'
   ou toute séquence dérivée de cette séquence SEQ ID NO : 10, notamment par suppression et/ou substitution et/ou addition d'un ou plusieurs nucléotides, ladite séquence dérivée s'hybridant, tout comme la séquence SEQ ID NO : 10, avec tout ou partie de la séquence nucléotidique complémentaire des séquences nucléotidiques délimitées par les nucléotides situés aux positions 9 et 38 des séquences SEQ ID NO : 1, 3, et 5,
- la séquence nucléotidique SIL8 représentée par la séquence SEQ ID NO : 11 suivante :
   5' CAGTCCCAAGTGCCTGAACACACATGCACC 3'
   ou toute séquence dérivée de cette séquence SEQ ID NO : 11, notamment par suppression et/ou substitution et/ou addition d'un ou plusieurs nucléotides, ladite séquence dérivée s'hybridant, tout comme la séquence SEQ ID NO : 11, avec tout ou partie des séquences nucléotidiques délimitées par les nucléotides situés aux positions 276 et 302 des séquences SEQ ID NO : 1, 3, et par les nucléotides situés aux positions 273 et 299, de la séquence SEQ ID NO : 5.

L'invention concerne également les couples d'amorces, dont chacune des deux amorces comprend, indépendamment l'une de l'autre, environ 10 à environ 30 nucléotides, caractérisés en ce qu'ils sont choisis de telle façon que l'une des deux séquences d'un couple d'amorces s'hybride avec une séquence d'environ 10 à environ 30 nucléotides comprise dans la séquence nucléotidique complémentaire de la séquence délimitée par les nucléotides situés aux positions 1 et environ 60 des séquences nucléotidiques SEQ ID NO : 1, 3, et 5, tandis que l'autre séquence de ce même couple s'hybride avec une séquence d'environ 10 à environ 30 nucléotides comprise entre le nucléotide situé en position 94 et le dernier des nucléotides des séquences SEQ ID NO : 1, 3, et 5.

L'invention a plus particulièrement pour objet les couples d'amorces pour l'amplification génique tels que définis ci-dessus, caractérisés en ce que :
- l'une des amorces est choisie parmi les séquences comprenant la séquence SIL10 représentée par SEQ ID NO : 10, ou toute séquence dérivée de cette dernière, telle que définie ci-dessus, ladite amorce étant avantageusement marquée, notamment de manière radioactive ou fluorescente,
- tandis que l'autre amorce est choisie parmi les séquences comprenant la séquence SIL8 représentée par SEQ ID NO : 11, ou toute séquence dérivée de cette dernière, telle que définie ci-dessus.

L'invention a également pour objet un procédé d'identification de populations ou races des mammifères ruminants tels que les bovins, les ovins, et les caprins, ledit procédé étant effectué à partir d'un échantillon biologique prélevé sur l'animal, notamment à partir de sperme, embryon, sang, lait, poils, carcasse, ou viande, ou autres produits dérivés de ces derniers, et permettant de contrôler, voire de certifier, l'appartenance ou la non-appartenance de l'animal sur lequel a été prélevé ledit échantillon biologique à une population ou race de mammifères ruminants, ce procédé comprenant :
- une étape d'amplification du nombre de copies des différentes formes allèliques du gène *SILVER,* à savoir des allèles *SI*, et/ou *si*, et/ou *si₁*, et/ou de fragments de ces formes allèliques, spécifiques d'une population ou race de mammifères ruminants déterminés, et susceptibles d'être présents dans ledit échantillon biologique,
- une étape de détection desdites formes allèliques ou fragments de ces dernières.

L'invention a plus particulièrement pour objet un procédé d'identification tel que défini ci-dessus, caractérisé en ce que l'étape d'amplification du nombre de copies des différentes formes allèliques du gène *SILVER,* ou des fragments de ces formes allèliques, est effectué à l'aide d'un couple d'amorces susmentionné.

L'invention concerne plus particulièrement un procédé d'identification tel que défini ci-dessus, caractérisé en ce que :
- la détection d'un génotype comprenant l'allèle *si* dans l'échantillon biologique étudié, permet de certifier que ledit échantillon provient d'un animal appartenant à la race charolaise ou ayant au moins un ascendant de race charolaise,
- la détection d'un génotype comprenant l'allèle *si₁* dans l'échantillon biologique étudié, permet de certifier que ledit échantillon provient d'un animal appartenant à la race simmental ou ayant au moins un ascendant de race simmental,
- la détection d'un génotype comprenant l'allèle *SI,* permet de certifier que ledit échantillon ne provient pas d'un animal de la race charolaise.

L'invention a également pour objet un kit pour la mise en oeuvre d'un procédé tel que défini ci-dessus, caractérisé en ce qu'il comprend au moins un couple d'amorces susmentionné, et le cas échéant les réactifs nécessaires à la mise en oeuvre de la réaction d'amplification du nombre de copies des différentes formes allèliques du gène *SIL VER.*

L'invention sera davantage illustrée à l'aide de la description détaillée qui suit de la mise en évidence du gène *SILVER* bovin, et de ses formes allèliques *si* et *si₁.*

### Légende des figures

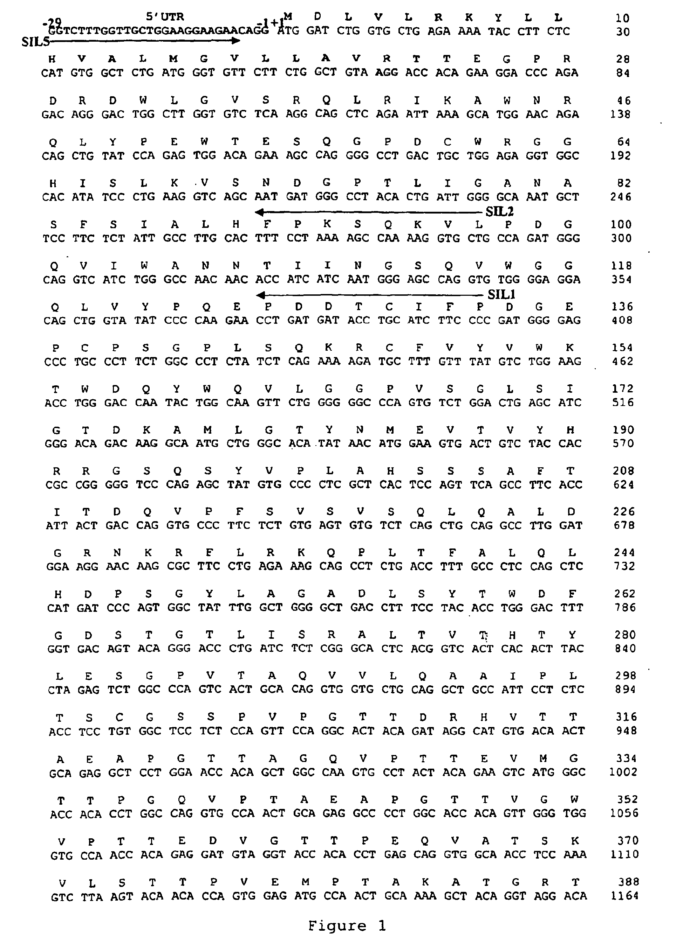

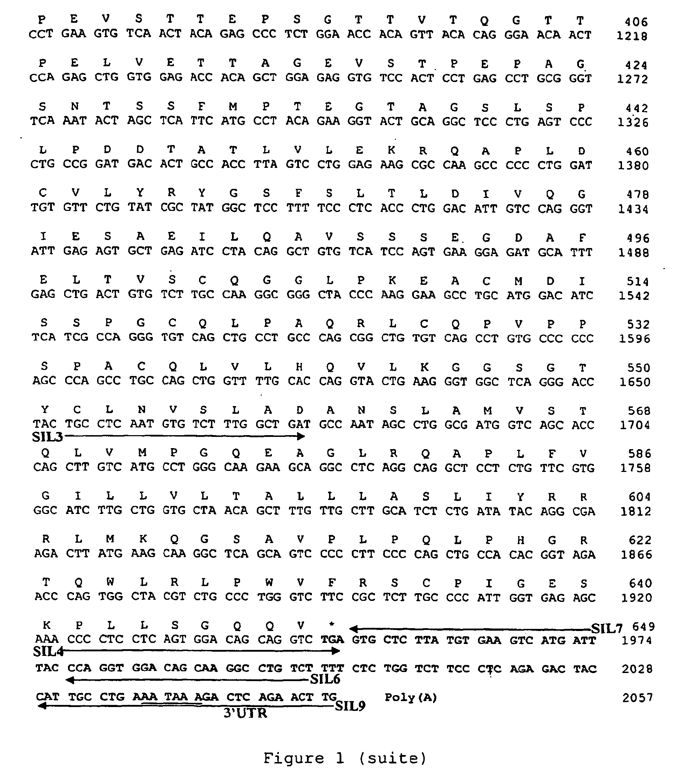

- Figure 1 : Transcrit du gène *SILVER* Bovin. La région 5' UTR est de 29 pb. La région 3'UTR a une taille de 107 pb suivie d'une queue polyA. Le site de polyadénylation est souligné. La protéine Pmel17 bovine est formée de 649 acides aminés. Le peptide signal est formé par les 24 premiers acides aminés. L'allèle *si* charolais conduit à la substitution d'une G glycine (G) en position 22 par une arginine (R).

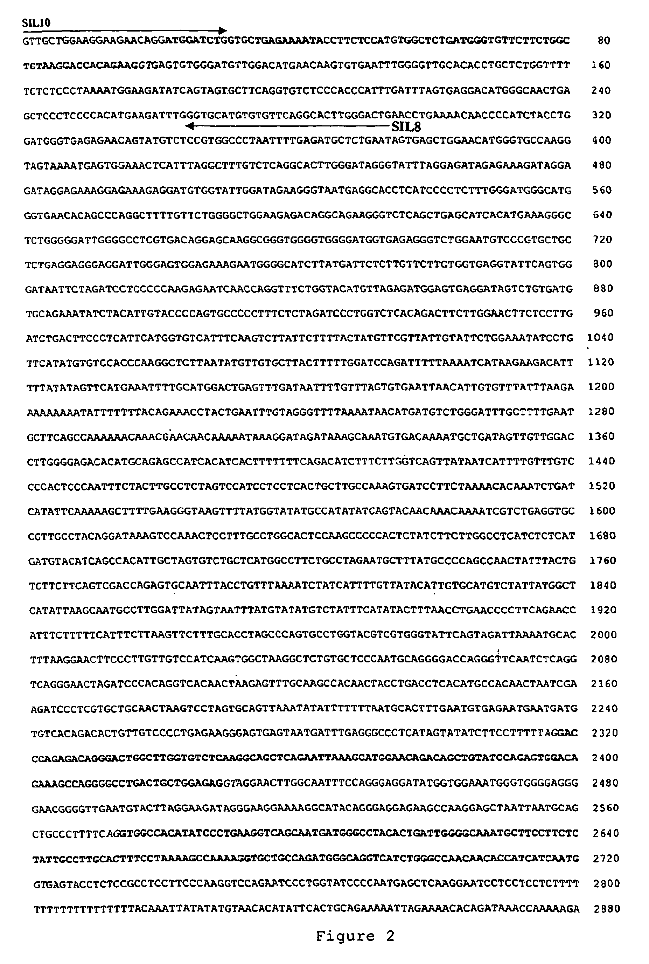

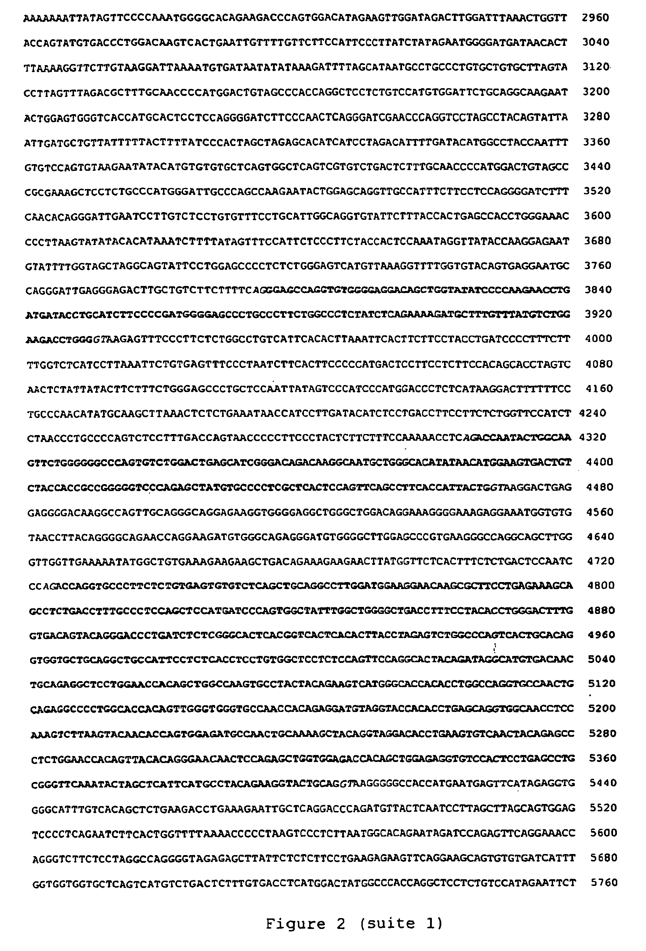

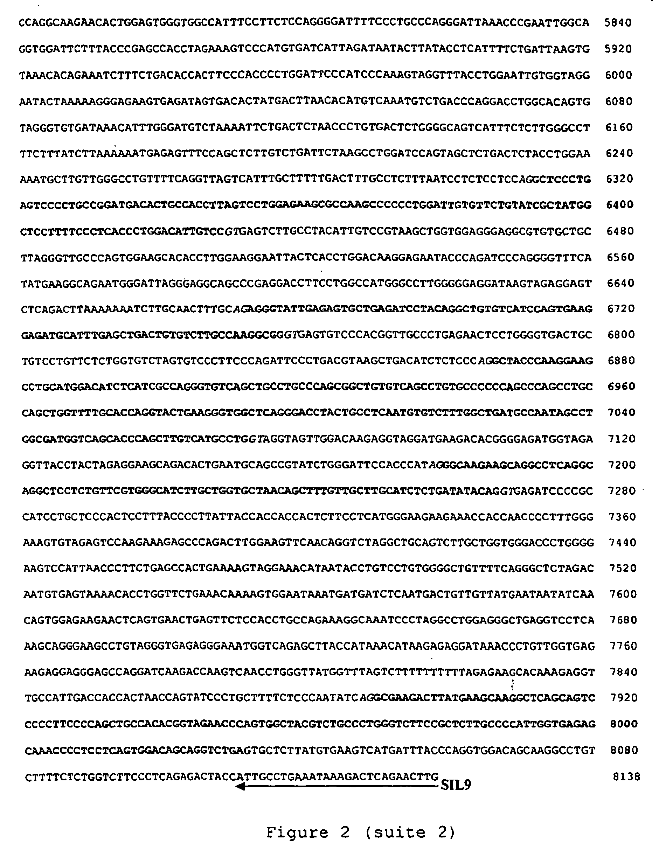

- Figure 2 : Structure génomique de la région codante du gène *SILVER* Bovin. Les amorces SIL10 et SIL9 qui ont permis de l'amplifier à partir de l'ADN génomique sont indiquées. Les différents exons sont en gras. Les coordonnées des exons au niveau de ce fragment d'ADN sont récapituléesdans le tableau 2. Les sites donneurs GT et les sites accepteurs AG de l'épissage sont en italiques. La mutation charolaise (substitution de la guanine par l'adénine) est en position 96 (premier exon).

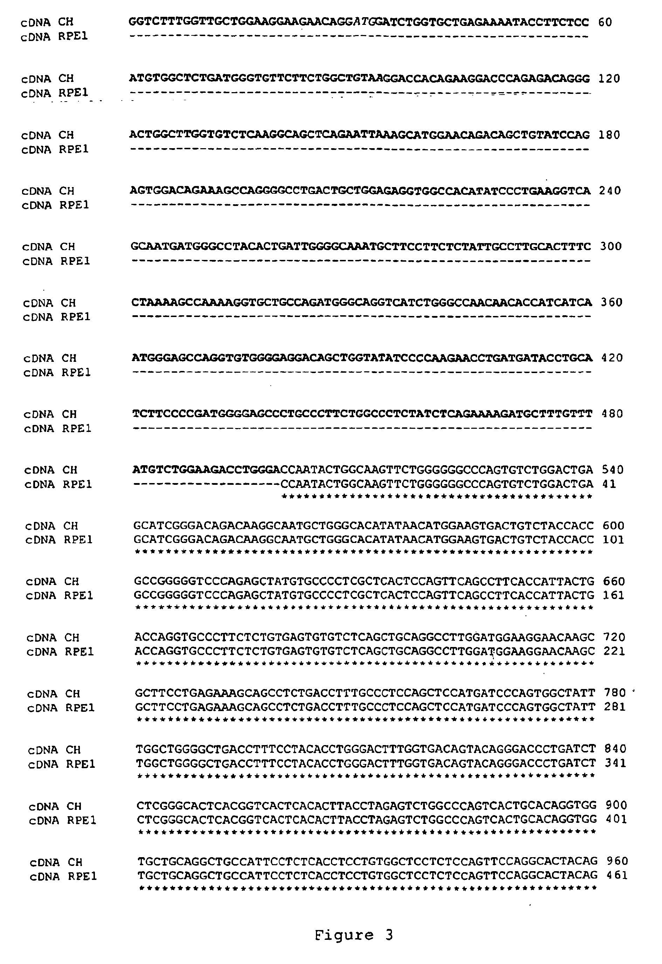

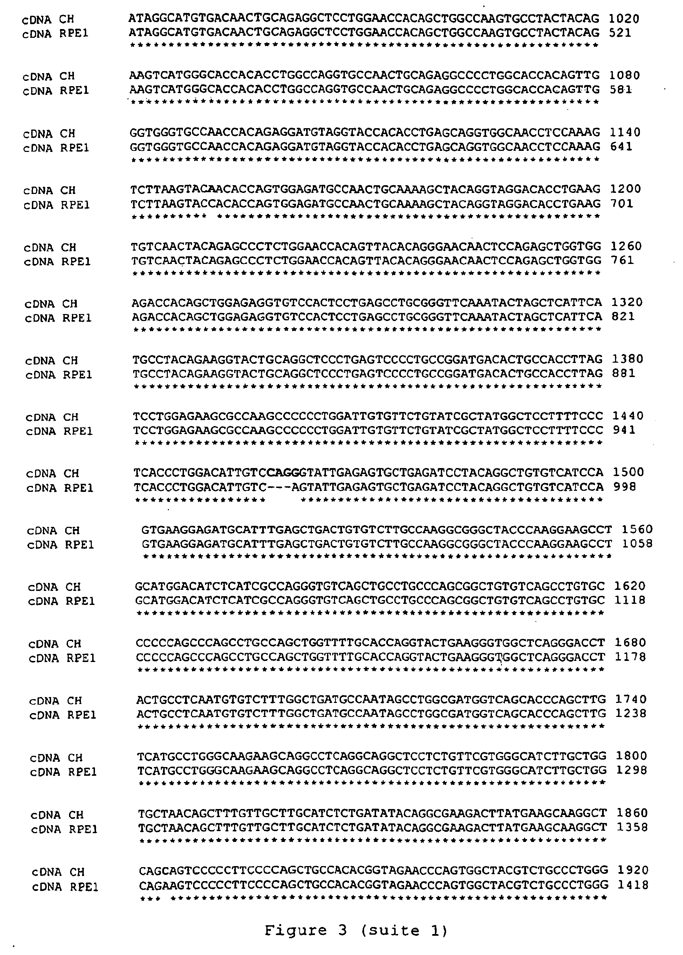

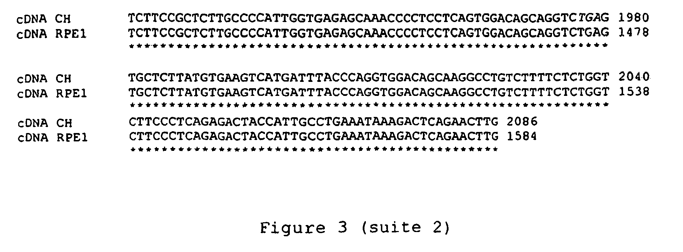

- Figure 3 : Comparaison des séquences nucléotidiques des transcrits du gène *SILVER* charolais et du transcrit RPE1. Les différences entre les deux séquences sont indiquées en gras. Il s'agit de l'absence de 499 pb de la région 5'mais également des modifications (A1151C), (CAG1458), (G1461A) et (C1864A). La mutation charolaise (G93A) est en position 93 (en gras et italique). Le codon d'initiation ATG de la traduction est en position 30 (en italiques) et le codon de terminaison (TGA) est en position 1977 (en italiques).

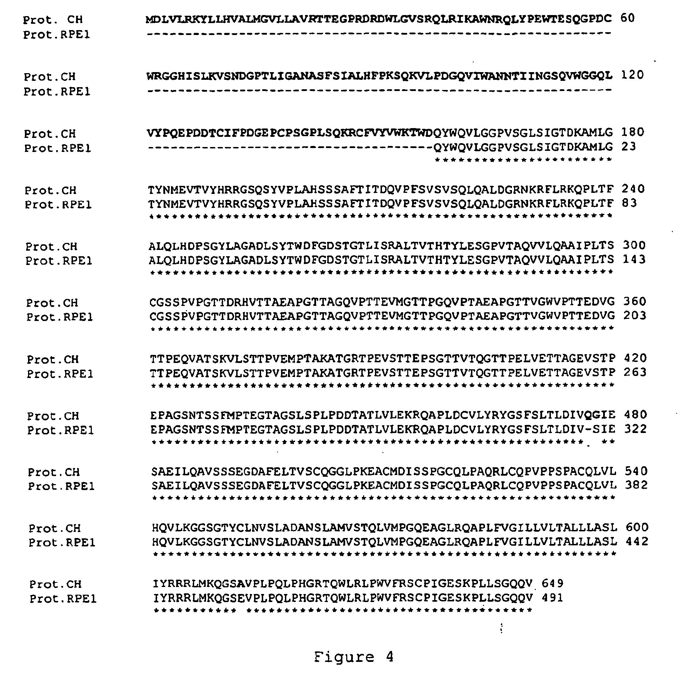

- Figure 4 : comparaison de séquences peptidiques déduites du transcrit du gène *SILVER* charolais et de celle déduite du transcrit RPE1. Les différences entre les deux séquences sont en gras. Il s'agit de l'absence des 157 premiers acides aminés mais également des modifications Q4677, G478S, et A612E. La modification (en gras et italique) qui caractérise la race charolaise est en position 22 (G22R).

### I) Matériel et méthodes

### Extraction des ARNs totaux à partir des échantillons de peaux bovines

L'extraction se pratique à partir d'un échantillon de peau de 2 cm² prélevé sur un individu bovin le plus rapidement possible après son abattage. La peau est rasée avec une lame de scalpel et débarrassée de sa couche adipeuse. L'échantillon est ensuite découpé en petits morceaux. L'extraction des ARNs est réalisée à l'aide du kit "RNeasy Maxi Kit" (Qiagen ; Référence : 75162) selon les recommandations du fournisseur. Les ARN totaux ainsi préparés sont utilisés pour la synthèse de l'ADN complémentaire (ADNc). Ils sont également utilisés dans la technique RACE-PCR pour disposer des régions 5' et 3' UTRs (régions non traduites) du transcrit du gène *SILVER.*

### Synthèse des ADNcs

5 µg des ARN totaux des race charolaise et salers ont été rétro-transcrits à l'aide de la reverse transcriptase SuperScriptII (Invitrogen ; référence 18064-014), selon les recommandations du fournisseur. Les ADN complémentaires ainsi obtenus sont utilisés comme matrice pour obtenir en particulier la partie codante du gène *SILVER.*

### Obtention des extrémités 5' et 3' d'ADN complémentaire

Le kit, SMART RACE cDNA Amplification Kit (Clontech ; référence K1811-1) a été utilisé pour obtenir des ADN complémentaires ligaturés à des adaptateurs en 5' et en 3'. 5 µg d'ARN totaux ont été utilisés selon les recommandations du fournisseur (Clontech).

### Purification de l'ADN génomique à partir des échantillons de sang

5 ml de sang prélevé sur EDTA, transféré dans un tube 50 ml (Nalgène) sont dilués avec 4 volumes de TE 20 :5 (Tris 20 mM : ETDA 5 mM). L'échantillon est ensuite incubé 15 min sur glace pour que la lyse des hématies ait lieu. Après une centrifugation de 4000 tpm pendant 20 min à 4°C le surnageant est éliminé doucement. Les cellules sont ensuite remises en suspension dans 15 ml de TE 20 : 5, suivi d'une centrifugation pendant 15 min à 4000 tpm et à 4°C. Deux lavages sont effectués (jusqu'à ce que le culot soit translucide). Le culot est ensuite repris dans ½ du volume de sang de départ avec du TE 20: 5, auquel on rajoute du SDS (1% final) et de la protéinase K (200 µg/ml final). Le mélange est incubé sous agitation (300 tpm) toute la nuit à 37 °C. L'ADN est ensuite précipité par addition de 1/3 du volume en acétate d'ammonium 7,5 M et 2 volumes d'éthanol absolu froid. Le culot est ensuite lavé avec de l'éthanol à 70%, séché, et repris (100 ng/µl) dans de l'eau et conservé à 4°C.

### Amplifications par la technique PCR

Les amplifications de fragments d'ADN comportent invariablement une phase de dénaturation, une phase d'hybridation et une phase d'élongation. Ce cycle est répété 35 fois et il est suivi d'une étape d'élongation de 7 min. La durée de chacune des phases ainsi que les amorces utilisées seront précisés pour chaque amplification.

### Purification de fragments d'ADN après séparation sur gel

Cette technique d'élution est utilisée dans les cas où il s'avère nécessaire de séparer un fragment d'intérêt de l'ADN contaminant, comme par exemple pour la préparation d'un insert avant clonage. Pour cela, on a utilisé le kit « QiaQuick Gel Extraction kit« (Qiagen, référence 28706) selon les recommandations du fabricant.

### Technique de clonage

Les inventeurs ont utilisé pour cloner les fragments d'ADN, deux types de vecteurs commerciaux (Invitrogen). Le vecteur pCR2.1-TOPO (référence K4500-01) pour le clonage de fragments d'ADN de taille inférieur à 2 kpb et le vecteur TOPO XL (référence K4750-20) pour le clonage de fragments de tailles supérieurs à 2 kpb. Le clonage s'effectue selon les recommandations du fournisseur.

### Séquençage de fragments d'ADN

Les séquences de fragments d'ADN clonés sont déterminées selon le principe de la technique de Sanger à l'aide d'un séquenceur automatique (ABI Prism 310 Genetic Analyser, Perkin Elmer).

Les réactions de séquençage sont menées grâce à l'utilisation d'une enzyme thermostable. Elles nécessitent environ 200 à 400 ng de matrice plasmidique, un mélange réactionnel commercial (Terminator Ready Mix, PRISM Ready Reaction Ampli *Taq* FS, Perkin Elmer) contenant les dNTPs, les ddNTPs, le MgCl₂ et l'ADN polymérase *(Taq* FS) ainsi que 15 pmol d'amorce. Vingt-cinq cycles sont réalisés dans un volume final de 20 µL : dénaturation à 96 °C 10 s, hybridation de l'amorce à 55 °C 5 s et élongation à 60 °C 4 min. L'ADN néosynthétisé est précipité par 80 µL d'isopropanol 75%, lavé par 250 µL d'isopropanol 75% afin d'éliminer les réactifs non utilisés qui pourraient perturber la migration, séché puis repris dans 20 µL de TSR avant d'être dénaturé par traitement thermique.

### II) Résultats

### Données expérimentales

### Isolement des régions 5' et 3' UTRs du gène SILVER de la race bovine charolaise

Les régions 5' et 3' UTRs (régions non traduites du transcrit) du gène *SIL VER* de la race charolaise ont été obtenues à l'aide de couples d'amorces (Fig. 1) (SIL1/UPM et SIL2/NUP) et (SIL3/UPM et SIL4/NUP) respectivement. Ces amorces ont été déduites à partir de régions conservées des gènes *SILVER* humain (ACC. AK092881) et murin (ACC. AK012808) disponibles dans les banques de données. Dans les deux cas une PCR dite nichée est nécessaire pour obtenir un produit d'amplification. 1,5 µl d'ADNc couplé aux adaptateurs ont été utilisés comme matrice. Nous avons utilisé la polymérase Abgene (Référence AM-266-615) selon les recommandations du fournisseur. Les conditions PCR sont les suivantes : 94°C 2min, (94°C 30, 61°C 30sec, 72°C 1min) X 35 cycles, 72°C 7min.

### Obtention de la région 5'UTR

Après une première amplification avec le couple d'amorce SIL1/UPM (UPM : 10X Universal Primer A Mix ; Clontech), une seconde PCR avec le couple d'amorces SIL2/NUP (NUP : Nested Universal Primer A ; Clontech) a été réalisée avec 1,5 µl de la première PCR dans les mêmes conditions d'amplification. L'amplifiat obtenu est ligaturé dans le vecteur pCR 2.1-TOPO et séquencé. La séquence obtenue comporte (Fig. 1) la région 5'UTR de 29 pb et 236 pb de la région codante du gène *SILVER.*

### Obtention de la région 3'UTR

La même procédure a été employée pour obtenir la région 3'UTR du gène en utilisant les couples d'amorces SIL3/UPM et SIL4/NUP en première et en deuxième PCR respectivement. L'amplifiat obtenu a été également ligaturé dans le vecteur pCR 2.1-TOPO et séquencé. La séquence obtenue comporte les 27 dernières paires de bases de la région codante (Fig. 1) suivie de la région 3'UTR d'une taille de 107 pb et se termine par une queue poly(A).

### Isolement de la totalité de la partie codante du gène SILVER

A partir des informations de séquence des régions 5' et 3' UTRs (Fig. 1) nous avons fait synthétiser trois amorces SIL5 (sens), SIL6 et SIL17 (anti-sens). Ces amorces ont été utilisées en première PCR (SIL5/SIL6) et en deuxième PCR (SIL5/SIL7) pour amplifier la totalité de la partie codante du gène *SILVER* à partir de l'ADNc des races charolaise et salers. 4 µl de chacun des amplifiats (charolais et salers) ont été clonés séparément dans le vecteur TOPO XL (Invitrogen) et séquencés. La figure 1 présente la totalité de la séquence obtenue pour la race charolaise.

### Découverte de l'allèle charolais

La comparaison des séquences codantes du gène *SIL VER* charolais et salers a révélé une seule différence : la substitution d'une guanine (Fig. 1) en position 64 chez le salers en une adénine chez le charolais. Nous avons désigné cet allèle du gène *SILVER* : *si.*

### Etablissement de la structure génomique de la partie codante du gène SILVER

Pour obtenir la totalité de l'information génétique contenant la partie codante du gène *SILVER* de la race charolaise, nous avons utilisé le couple d'amorces SIL5/SIL9 (Fig. 1). En utilisant une Taq polymerase (Expand Long Template PCR System, Roche, référence 1 681 834) qui permet d'amplifier des fragments d'ADN jusqu'à 20 kpb, nous avons obtenu un fragment d'ADN génomique charolais d'environ 8 kpb. Ce dernier a été cloné dans le vecteur TOPO XL (Invitrogen) et entièrement séquencé. La figure 2 représente la structure exon/intron de la partie codante du gène *SILVER* charolais.

### Génotypage de l'allèle si

Pour l'étude de cette région, nous avons fait synthétiser une amorce (SIL8) dans la première région intronique (Fig. 2). En utilisant le couple d'amorce SIL10/SIL8, on obtient un fragment d'ADN génomique de 294 pb. Le séquençage direct de l'amplifiat nous renseigne immédiatement sur la situation de la mutation charolaise dans les autres races. Nous avons également analysé le cas de certains croisés dont l'un des parents est un individu charolais. Le tableau 1 ci-après présente les résultats du génotypage de allèle *si* et pour différentes races bovines.

**Tableau 1 : Génotypage de l'allèle si et si₁ de différentes races bovines. L'allèle si est caractéristique de tous les individus de la race Charolaise. Cinq animaux croisés dont l'un des parents est charolais ont été également génotypés pour l'allèle si. Un des croisés est hétérozygote si/si₁. Il porte l'allèle charolais si et l'allèle si₁. L'allèle si₁ caractérise les individus d'origine Simmental.**

| Races bovines | | Allèles du gène *SIL VER* | | | | | Nombre d'individus testés |
|---|---|---|---|---|---|---|---|
| | | *si*/*si* | *SI*/*SI* | *SI*/*si* *si₁*/*si₁* | *si*/*si₁* | *SI*/*si₁* | |
| Charolaise | | + | - | - | - | - | 41 |
| | | - | | | | | |
| Limousine | | - | + | - | - | - | 11 |
| | | - | | | | | |
| Blonde | | - | + | - | - | - | 5 |
| d'Aquitaine | | - | | | | | |
| Salers | | - | + | - | - | - | 8 |
| | | | | | | | |
| Maine Anjou | | - | + | - | - | - | 3 |
| | | - | | | | | |
| Montbéliarde | | - | + | - | - | - | 3 |
| | | - | | | | | |
| Gasconne | | - | + | - | - | - | 12 |
| | | - | | | | | |
| Aubrac | | - | + | - | - | - | 10 |
| | | - | | | | | |
| Parthenaise | | - | + | - | - | - | 3 |
| | | - | | | | | |
| Bazadaise | | - | + | - | - | - | 6 |
| | | - | | | | | |
| Normande | | - | + | - | - | - | 10 |
| | | - | | | | | |
| Blanc bleue | | - | + | - | - | - | 6 |
| | | - | | | | | |
| Prim'Holstein | | - | + | - | - | - | 1 |
| | | - | | | | | |
| Croisés | 4 | - | - | + | - | - | |
| | | - | | | | | 5 |
| Charolaise | 1 | - | - | - | + | - | |
| | | - | | | | | |
| Simmental | 2 | - | + | - | - | - | |
| | | - | | | | | 12 |
| 5 | | - | - | - | - | + | |
| française | 5 | - | | | | | |
| | | - | - | - | - | - | |
| | | + | | | | | |

### III) Analyse des données expérimentales

### Transcrit et produit du gène SIL VER bovin

L'ARNm du gène *SIL VER* bovin a une taille de 2086 pb. Les région 5' et 3' UTRs ont une taille de 29 pb et 107 pb respectivement (Fig.1). Le cadre de lecture ouvert a une taille de 1950 pb et code une protéine de 649 acides aminés avec un peptide signal de 24 acides aminés (Fig. 1).

### Structure génomique de la partie codante

La partie codante du gène *SILVER* bovin comporte 11 exons et 10 introns (Fig.2). Les taille et la position des différents exons sont mentionnées dans le tableau 2 ci-après. Les sites donneurs (GT) et accepteurs (AG) de l'épissage sont parfaitement conservés.

**Tableau 2 : Positions des exons codants au niveau génomique et au niveau du transcrit du gène SILVER Bovin. La position du premier nucléotide au niveau génomique correspond au premier nucléotide 5' de l'amorce SIL10 (Fig. 2). Au niveau du transcrit le premier nucléotide correspond au démarrage de la transcription (Fig. 1).**

| Exons codants | Coordonnées au niveau | | Tailles pb |
|---|---|---|---|
| | Génomique | Transcrit | |
| Exon1 | 22-97 | 30-105 | 76 |
| Exon2 | 2318-2428 | 106-216 | 111 |
| Exon3 | 2574-2720 | 217-363 | 147 |
| Exon4 | 3796-3930 | 364-498 | 135 |
| Exon5 | 4307-4468 | 499-660 | 162 |
| Exon6 | 4725-5405 | 661-1341 | 681 |
| Exon7 | 6313-6429 | 1342-1458 | 117 |
| Exon8 | 6673-6757 | 1459-1543 | 85 |
| Exon9 | 6867-7072 | 1544-1749 | 206 |
| Exon10 | 7180-7267 | 1750-1837 | 88 |
| Exon11 | 7890-8031 | 1838-1979 | 142 |

### Appartenance raciale de l'allèle si et la découverte d'un nouvel allèle

L'analyse des données du génotypage (tableau 1) démontre que l'allèle ***si*** à l'état homozygote est rencontré uniquement pour les individus appartenant à la race charolaise. Les individus croisés de première génération dont l'un des parents est charolais sont également identifiables par leur génotype hétérozygote *(**si***/***SI***)*.*

Un des croisés (tableau 1) que nous avons analysé présente le génotype suivant : ***si*/*si₁***. Il porte l'allèle charolais et un nouvel allèle ***si₁***. L'allèle ***si₁***, se caractérise (Fig. 1) par la délétion de trois nucléotides T, T, et C en position 53, 54 et 55 respectivement (ΔT53, ΔT54 et ΔC55). Les positions sont indiquées par rapport au premier nucléotide (adénine A) du démarrage de la traduction. Cette délétion engendre (Fig.1) la perte d'un acide aminé leucine en position 18 (ΔL18). Du fait de la position de cette délétion (adjacente à la mutation charolaise) en plus du rôle de l'acide aminé (leucine) dans l'efficacité de l'élimination du peptide signal, ce deuxième allèle du gène *SILVER* doit avoir un effet similaire à l'allèle charolais : une dilution de la couleur de la robe. Une des races, si non la seule qui possède un effet de dilution similaire à celui du charolais est la race Simmental.

### Génotypage de l'allèle si₁

Nous avons génotype 12 individus appartenant à la race Simmental française. Les résultats obtenus sont présentés dans le tableau 1. L'allèle ***si₁*** est rencontré dans la race simmental française à l'état homozygote (***si₁***/***si₁***) et à l'état hétérozygote (***si**₁*/***SI***). L'allèle sauvage à l'état homozygote (*SI*/*SI*) est également rencontré.

### Homologies avec les données disponibles dans les banques

### 1. Données humaines et murines

La protéine Silver bovine (nommée également Pmel17) présente 79% et 76% d'homologie avec ses homologues humain (ACC. AK092881) et murin (ACC. AK092881) respectivement. Il est à noter que l'acide aminé Glycine (G) qui se trouve substitué par une arginine (R) dans le cas de la race bovine charolaise (Fig.1) est conservé chez les autres bovins, l'homme et la souris.

### 2. Données bovines

La comparaison de la séquence du transcrit du gène *SILVER* bovin avec les données disponibles dans les banques révèle la présence d'informations de séquences du gène *SILVER* bovin. Ces séquences sont de deux types : des ESTs (ACC : BM106313; AW352955 ; AW478070 ; BF604634; BF599555), mais également une séquence partielle (ACC : M81193) du transcrit du gène *SILVER* bovin.

Cette séquence partielle a été publiée sous le nom de RPE1 (retinal pigment epithelium) en 1992 par Kim RY et Wistow GJ (The cDNA RPE1 and monoclonal antibody HMB-50 define gene products preferentially expressed in retinal pigment epthelium, Exp Eye Res 1992 Nov ; 55 (5) : 657-62). Elle a été isolée à partir de l'épithélium rétinien. La séquence RPE1 (Fig. 3) publiée par Kim et Wiston correspond probablement à une séquence partielle du transcrit du gène *SILVER* bovin ou à un isoforme du même gène, exprimé au niveau de la rétine. Il lui manque (Fig. 3) en effet 499 pb de la région 5', région où se trouve justement la mutation charolaise. La séquence diffère également de celle que nous avons identifié pour le gène *SILVER* par : la substitution de l'adénine en position 1151 par une cytosine (A1151C) ; la délétion du codon CAG en position 1458 (ΔCAG1458) ; la substitution de la guanine en position 1461 par une adénine (G1461A) et la substitution de la cytosine en position 1864 par une adénine (C1864A). Les positions sont citées par rapport au transcrit bovin charolais (Fig. 3).

Ces différences au niveau de la séquence nucléotidique se traduisent par des modifications au niveau peptidique (Fig. 4). La délétion ΔCAG1458 conduit à la délétion de l'acide aminé glutamine en position 477 (ΔQ477) ; la substitution G1461A au remplacement de l'acide aminé glycine par l'acide aminé sérine en position 478 (G478S) et la substitution C1864A au changement de l'acide aminé alanine en position 612 par l'acide glutamique (A612E). La substitution A1151C est silencieuse. La position des acides aminés est citée par rapport à la séquence peptidique que nous avons déterminée (Fig. 4).

### III) Commentaire et Discussion

La mutation *si* identifiée constitue le premier et unique marqueur moléculaire et génétique caractérisant une seule race bovine en l'occurrence la race charolaise à l'exclusion de toutes les autres races. Sa découverte résulte de l'analyse des données phénotypiques et moléculaires obtenues chez la souris et l'homme publiées par d'autres auteurs (Berson et al., Mol Biol Cell 2001 12(11):3451-64 ; Raposo et al., J Cell Biol. 2001 Feb 19;152 (4):809-24 ; Kwon et al., Nucleic Acids Res. 1995 Jan 11, 23(1):154-8 ; Kwon et al Proc Natl Acad Sci USA. 1991 Oct 15; 88(20):9228-32 ; Kim et al,. 1992, Exp Eye Res Nov;55(5):657-62.). Elle prend en compte les travaux concernant la structure des mélanosomes chez les bovins (Renieri et al., Pigment Cell Res. (1993), 6, 165-170). Elle est également basée sur une réévaluation des données de séquences murines, humaines et bovines disponibles dans les banques. Elle est aussi l'aboutissement d'un travail expérimental spécifique exploitant le génome des races bovines sélectionnées par les professionnels sur la base de leur phénotype de coloration de la robe.

Le marqueur génétique, sujet de cette innovation, caractérise l'allèle du gène bovin *SILVER* (nommé également *PMEL 17, GP100 ou ME20M*). Le gène *silver* de la souris participe à la biogenèse du mélanosome et cela est également probable chez les autres mammifères. La mutation du gène bovin *PMEL17 (SILVER)* identifie ce dernier comme le gène de dilution, nommé ainsi précédemment sur la base des nombreuses observations découlant de l'analyse des phénotypes d'animaux croisés, l'un des parents étant un charolais de race pure (inscrit au Herd Book).

L'allèle ***si*** charolais du gène *PMEL17* est caractérisé par la substitution de la guanine en (Fig.1) position 64 par une adénine (G64A). Cette substitution modifie le codon GGG en position 22, spécifiant l'acide aminé glycine ou G, en un codon AGG spécifiant l'arginine ou R (le nucléotide n°1 correspond à l'adénine du codon de démarrage ATG qui spécifie la méthionine ou M ; Fig. 1).

La mutation charolaise G64A affecte le peptide signal de la protéine PMEL 17, en un site essentiel pour sa maturation correcte dans le réticulum endoplasmique du mélanocyte. Ainsi, la protéine PMEL 17 incorrectement maturée ne peut plus s'engager dans la voie normale de son adressage au mélanosome via l'appareil de Golgi, organelle au sein duquel s'effectue la glycosylation. La PMEL 17 mutée ne peut donc pas atteindre finalement sous une forme correcte le mélanosome où sa fonction normale est de participer à la matrice sur laquelle s'effectue la polymérisation des mélanines et leur fixation. Le mélanosome charolais n'est donc pas ou très peu coloré, en raison du défaut structural ou de l'absence de la protéine PMEL17. A l'état homozygote, les deux allèles du gène *SILVER* sont mutés, ce qui a pour conséquence que toutes les protéines PMEL17 sont inefficaces dans la polymérisation et le dépôt des mélanines. Ainsi la mutation G64A conduit à la formation de la couleur « blanc crémeux », typique du bovin charolais. A l'état hétérozygote, un seul allèle est muté ce qui entraîne probablement que seule une moitié des protéines PMEL17 est inefficace. Le défaut partiel de la matrice du mélanosome se manifeste par une dilution de la couleur de l'animal croisé.

Dans le cas du deuxième allèle ***si₁**,* il s'agit de la délétion de trois nucléotides (ΔT53, ΔT54 et ΔC55) dans la région codant le peptide signal. Cet allèle ***si**₁* conduit précisément à la délétion d'un acide aminé hydrophobe (leucine). Du fait de la position de cette délétion (adjacente à la mutation charolaise) en plus du rôle de l'acide aminé (leucine) dans l'efficacité de l'élimination du peptide signal, ce deuxième allèle du gène *SILVER* aura un effet similaire à l'allèle charolais : une dilution de la couleur de la robe. Nous avons génotype 12 individus de la race simmental française. 5 individus sont homozygotes ***si₁***/***si₁**,* 5 autres sont hétérozygotes ***SI***/***si₁*** et deux possèdent l'allèle sauvage à l'état homozygote (***SI*/*SI***).

La race simmental a le même ancêtre commun datant du moyen age et dont le berceau se trouve dans la vallée de la rivière «Simme» en suisse, dans l'Oberland bernois (canton de Berne).

L'hétérozygotie que présente la race simmental française n'est pas surprenante dans la mesure ou elle fait partie des races laitières en France qui sont très ouvertes sur l'extérieur, où la proportion de gènes étrangers atteint ou dépasse 75% (Boichard et al. 1996, INRA Prod. Anim., 9 (5), 323-335).

## Revendications

1. Utilisation, de séquences nucléotidiques du gène *SILVER* bovin représenté par SEQ ID NO : 1, codant pour la protéine bovine SI représentée par SEQ ID NO : 2, et des différentes formes allèliques de ce gène, ou de fragments de ce gène ou de ses différentes formes allèliques, pour la mise en oeuvre d'un procédé d'identification des différentes populations ou races bovines, ou de différents troupeaux bovins regroupant chacun plusieurs populations ou races bovines.

2. Utilisation de séquences nucléotidiques selon la revendication 1, pour la mise en oeuvre d'une méthode permettant de contrôler, voire de certifier, l'appartenance d'un animal à une population ou race bovine particulière, ou à un troupeau bovin particulier, ou, au contraire, permettant de certifier l'exclusion de cet animal de cette population ou race, ou de ce troupeau particulier.

3. Utilisation de séquences nucléotidiques selon la revendication 1 ou 2, **caractérisée en ce que** les populations ou races bovines ou troupeaux bovins sont d'origine française.

4. Utilisation selon l'une des revendications 1 à 3:
- de la séquence nucléotidique de la forme allèlique *si* représentée par SEQ ID NO : 3, codant pour la protéine bovine si représentée par SEQ ID NO : 4, ou à des fragments de cette forme allèlique comprenant la mutation G93A par rapport au gène *SI,* pour la mise en oeuvre d'une méthode permettant de contrôler, voire de certifier, l'appartenance d'un animal à la race charolaise,
- de la séquence nucléotidique de la forme allèlique ***si**₁* représentée par SEQ ID NO : 5, codant pour la protéine bovine si₁ représentée par SEQ ID NO : 6, ou de fragments de cette forme allèlique comprenant une délétion des trois nucléotides TTC situés aux positions 82, 83 et 84 par rapport au gène *SI,* pour la mise en oeuvre d'une méthode permettant de contrôler, voire de certifier, l'appartenance d'un animal à la race simmental,
- de la séquence nucléotidique du gène *SI* bovin représenté par SEQ ID NO : 1, ou à des fragments de ce gène, pour la mise en oeuvre d'une méthode permettant de certifier l'exclusion d'un animal de la race charolaise.

5. Utilisation selon l'une des revendications 1 à 4, de fragments des séquences nucléotidiques des formes allèliques *SI, si,* et *si₁*, représentées respectivement par SEQ ID NO : 1, 3, et 5, lesdits fragments étant choisis parmi ceux d'environ 10 à 300 nucléotides contenant les nucléotides situés aux positions 82 à 93 desdites séquences.

6. Utilisation selon l'une des revendications 1 à 5, de fragments des séquences nucléotidiques des formes allèliques *SI, si,* et *si₁*, lesdits fragments étant choisis parmi ceux de 294 nucléotides délimités par les nucléotides situés aux positions 9 et 302 des séquences SEQ ID NO : 1 et 3, ces fragments étant représentés respectivement par les séquences SEQ ID NO : 7, et SEQ ID NO : 8, et le fragment de 291 nucléotides délimité par les nucléotides situés aux positions 9 et 299 de la séquence SEQ ID NO : 5, ce fragment étant représenté par la séquence SEQ ID NO : 9.

7. Utilisation d'amorces nucléotidiques pour la mise en oeuvre d'un procédé d'identification des différentes populations ou races des mammifères ruminants tels que les bovins, par amplification du nombre de copies du gène *SILVER,* ou des différentes formes allèliques de ce gène, ou des fragments de ce gène ou de ses différentes formes allèliques, tels que définis dans l'une des revendications 1 à 6.

8. Utilisation d'amorces nucléotidiques selon la revendication 7, sous forme de couples d'amorces 5'-3', ces couples étant tels que :
- l'amorce 5' est choisie parmi l'amorce SIL10 représentée par la séquence SEQ ID NO : 10 suivante :
5' GTTGCTGGAAGGAAGAACAGGATGGATCTG 3'
ou toute séquence dérivée de cette séquence SEQ ID NO : 10, notamment par suppression et/ou substitution et/ou addition d'un ou plusieurs nucléotides, ladite séquence dérivée s'hybridant, tout comme la séquence SEQ ID NO : 10, avec tout ou partie de la séquence nucléotidique complémentaire des séquences nucléotidiques délimitées par les nucléotides situés aux positions 9 et 38 des séquences SEQ ID NO : 1, 3, et 5,
- l'amorce 3' est choisie parmi l'amorce SIL8 représentée par la séquence SEQ ID NO : 11 suivante :
5' CAGTCCCAAGTGCCTGAACACACATGCACC 3'
ou toute séquence dérivée de cette séquence SEQ ID NO : 11, notamment par suppression et/ou substitution et/ou addition d'un ou plusieurs nucléotides, ladite séquence dérivée s'hybridant, tout comme la séquence SEQ ID NO : 11, avec tout ou partie des séquences nucléotidiques délimitées par les nucléotides situés aux positions 276 et 302 des séquences SEQ ID NO : 1, 3, et par les nucléotides situés aux positions 273 et 299, de la séquence SEQ ID NO : 5.

9. Séquence nucléotidique **caractérisée par** le gène *SI* bovin représenté par SEQ ID NO : 1, ou par les fragments suivants du gène *SI:*
- tout fragment choisi parmi ceux de 10 à 300 nucléotides contenant les nucléotides situés aux positions 82 à 93 de la séquence SEQ ID NO : 1,
- le fragment SEQ ID NO : 7 de 294 nucléotides délimité par les nucléotides situés aux positions 9 et 302 de la séquence SEQ ID NO : 1.

10. Séquence nucléotidique **caractérisée par** le gène *si* bovin représenté par SEQ ID NO : 3, ou par les fragments suivants du gène *si* :
- tout fragment choisi parmi ceux de 10 à 300 nucléotides contenant les nucléotides situés aux positions 82 à 93 de la séquence SEQ ID NO : 3,
- le fragment SEQ ID NO : 8 de 294 nucléotides délimité par les nucléotides situés aux positions 9 et 302 de la séquence SEQ ID NO : 3.

11. Séquence nucléotidique **caractérisée par** le gène *si₁* bovin représenté par SEQ ID NO : 5, ou par les fragments suivants du gène *si₁* :
- tout fragment choisi parmi ceux de 10 à 300 nucléotides contenant les nucléotides situés aux positions 82 à 93 de la séquence SEQ ID NO : 5,
- le fragment SEQ ID NO : 9 de 291 nucléotides délimité par les nucléotides situés aux positions 9 et 299 de la séquence SEQ ID NO : 5.

12. Séquence nucléotidique **caractérisée en ce qu'**elle comprend :
- la séquence nucléotidique SIL10 représentée par la séquence SEQ ID NO : 10 suivante :
5' GTTGCTGGAAGGAAGAACAGGATGGATCTG 3'
la dite séquence SEQ ID NO : 10 s'hybridant avec tout ou partie de la séquence nucléotidique complémentaire des séquences nucléotidiques délimitées par les nucléotides situés aux positions 9 et 38 des séquences SEQ ID NO : 1, 3, et 5,
- la séquence nucléotidique SIL8 représentée par la séquence SEQ ID NO : 11 suivante :
5' CAGTCCCAAGTGCCTGAACACACATGCACC 3'
la dite séquence SEQ ID NO : 11 s'hybridant avec tout ou partie des séquences nucléotidiques délimitées par les nucléotides situés aux positions 276 et 302 des séquences SEQ ID NO : 1, 3, et par les nucléotides situés aux positions 273 et 299, de la séquence SEQ ID NO : 5.

13. Couples d'amorces, dont chacune des deux amorces comprend, indépendamment l'une de l'autre, de 10 à 30 nucléotides, **caractérisés en ce qu'**ils sont aptes à amplifier d'une façon spécifique le gène *SILVER* bovin représenté par SEQ ID NO : 1 et sont choisis de telle façon que l'une des deux séquences d'un couple d'amorces s'hybride avec une séquence de 10 à 30 nucléotides comprise dans la séquence nucléotidique complémentaire de la séquence délimitée par les nucléotides situés aux positions 1 et 60 des séquences nucléotidiques SEQ ID NO : 1, 3, et 5, tandis que l'autre séquence de ce même couple s'hybride avec une séquence de 10 à 30 nucléotides comprise entre le nucléotide situé en position 94 et le dernier des nucléotides des séquences SEQ ID NO : 1,3, et 5.

14. Couples d'amorces pour l'amplification génique selon la revendication 10, **caractérisé en ce que** :
- l'une des amorces est choisie parmi les séquences comprenant la séquence SIL10 représentée par SEQ ID NO : 10, selon la revendication 12, ladite amorce étant avantageusement marquée, notamment de manière radioactive ou fluorescente,
- tandis que l'autre amorce est choisie parmi les séquences comprenant la séquence SIL8 représentée par SEQ ID NO : 11, selon la revendication 12.

15. Procédé d'identification de populations ou races des mammifères ruminants tels que les bovins, les ovins, et les caprins, ledit procédé étant effectué à partir d'un échantillon biologique prélevé sur l'animal, notamment à partir de sperme, embryon, sang, lait, poils, carcasse, ou viande, ou autres produits dérivés de ces derniers, et permettant de contrôler, voire de certifier, l'appartenance ou la non-appartenance de l'animal sur lequel a été prélevé ledit échantillon biologique à une population ou race de mammifères ruminants, ce procédé comprenant :
- une étape d'amplification du nombre de copies des différentes formes allèliques du gène *SILVER,* à savoir des allèles *SI* représenté par SEQ ID NO : 1, et/ou *si* représenté par SEQ ID NO : 3, et/ou *si₁* représenté par SEQ ID NO : 5, et/ou de fragments de ces formes allèliques, spécifiques d'une population ou race de mammifères ruminants déterminés, et susceptibles d'être présents dans ledit échantillon biologique,
- une étape de détection desdites formes allèliques ou fragments de ces dernières.

16. Procédé d'identification selon la revendication 15, **caractérisé en ce que** l'étape d'amplification du nombre de copies des différentes formes allèliques du gène *SILVER,* ou des fragments de ces formes allèliques, est effectué à l'aide d'un couple d'amorces selon la revendication 13 ou 14.

17. Procédé d'identification selon la revendication 15 ou 16, **caractérisé en ce que** :
- la détection d'un génotype comprenant l'allèle *si* dans l'échantillon biologique étudié, permet de certifier que ledit échantillon provient d'un animal appartenant à la race charolaise ou ayant au moins un ascendant de race charolaise,
- la détection d'un génotype comprenant l'allèle *si₁* dans l'échantillon biologique étudié, permet de certifier que ledit échantillon provient d'un animal appartenant à la race simmental ou ayant au moins un ascendant de race simmental,
- la détection d'un génotype comprenant l'allèle *SI*, permet de certifier que ledit échantillon ne provient pas d'un animal de la race charolaise.

18. Kit pour la mise en oeuvre d'un procédé selon l'une des revendications 15 à 17, **caractérisé en ce qu'**il comprend au moins un couple d'amorces selon la revendication 13 ou 14 et le cas échéant les réactifs nécessaires à la mise en oeuvre de la réaction d'amplification du nombre de copies des différentes formes allèliques du gène *SIL VER.*

## Claims

1. Use of nucleotide sequences corresponding to the bovine *SILVER* gene represented by SEQ ID NO: 1, coding for the bovine SI protein represented by SEQ ID NO: 2, and to the different allelic forms of this gene, or corresponding to fragments of this gene or of its different allelic forms, for the implementation of a method for the identification of different cattle populations or breeds, or of different cattle herds each including several cattle populations or breeds.

2. Use of nucleotide sequences according to claim 1, for the implementation of a method making it possible to check, or even to certify, that an animal belongs to a particular cattle population or breed, or to a particular cattle herd, or, on the other hand, making it possible to certify the exclusion of this animal from this population or breed, or from this particular herd.

3. Use of nucleotide sequences according to one of claims 1 or 2, **characterized in that** cattle populations or breeds or cattle herds are of French origin.

4. Use according to one of claims 1 to 3,
- of the nucleotide sequence corresponding to the *si* allelic form represented by SEQ ID NO: 3, coding for the bovine si protein represented by SEQ ID NO: 4, or corresponding to fragments of this allelic form, said allelic form comprising the G93A mutation with respect to the *SI* gene, for the implementation of a method making it possible to check, or even to certify, that an animal belongs to the Charolais breed,
- of the nucleotide sequence corresponding to the *si₁* allelic form, represented by SEQ ID NO: 5, coding for the bovine *si₁* protein represented by SEQ ID NO: 6, or corresponding to fragments of this allelic form, said allelic form comprising a deletion of the three nucleotides TTC situated in positions 82, 83 and 84 with respect to the *SI* gene, for the implementation of a method making it possible to check, or even to certify, that an animal belongs to the Simmental breed,
- of the nucleotide sequence corresponding to the bovine *SI* gene represented by SEQ ID NO: 1, or to fragments of this gene, for the implementation of a method making it possible to certify the exclusion of an animal from the Charolais breed.

5. Use according to one of claims 1 to 4, of fragments of the nucleotide sequences corresponding to the allelic forms, *SI, si,* and *si₁,* represented by SEQ ID NO: 1, 3, and 5 respectively, said fragments being chosen from those of 10 to 300 nucleotides containing the nucleotides situated in positions 82 to 93 of said sequences.

6. Use according to one of claims 1 to 5, of fragments of the nucleotide sequences corresponding to the allelic forms *SI, si,* and *si₁,* said fragments being chosen from those of 294 nucleotides delimited by the nucleotides situated in positions 9 and 302 of the sequences SEQ ID NO: 1 and 3, these fragments being represented by the sequences SEQ ID NO: 7, and SEQ ID NO: 8 respectively, and the fragment of 291 nucleotides delimited by the nucleotides situated in positions 9 and 299 of the sequence SEQ ID NO: 5, this fragment being represented by the sequence SEQ ID NO: 9.

7. Use of nucleotide primers making it possible to amplify the number of copies of the SILVER gene, or of the different allelic forms of this gene, or of the fragments of this gene or of its different allelic forms, as defined in one of claims 1 to 6, for the implementation of a method for the identification of different populations or breeds of ruminant mammals such as cattle.

8. Use of nucleotide primers according to claim 7, in the form of 5'-3' primer pairs, these pairs being such that:
- the 5' primer is chosen from the SIL10 primer represented by the following sequence SEQ ID NO: 10:
5' GTTGCTGGAAGGAAGAACAGGATGGATCTG 3'
or any sequence derived from this sequence SEQ ID NO: 10, in particular by suppression and/or substitution and/or addition of one or more nucleotides, said derived sequence being hybridized, like the sequence SEQ ID NO: 10, with all or part of the nucleotide sequence complementary to the nucleotides sequences delimited by the nucleotides situated in positions 9 and 38 of the sequences SEQ ID NO: 1, 3, and 5,
- the 3' primer is chosen from the SIL8 primer represented by the following sequence SEQ ID NO: 11:
5'CAGTCCCAAGTGCCTGAACACACATGCACC3'
or any sequence derived from this sequence SEQ ID NO: 11, in particular by suppression and/or substitution and/or addition of one or more nucleotides, said derived sequence being hybridized, like the sequence SEQ ID NO: 11, with all or part of the nucleotide sequences delimited by the nucleotides situated in positions 276 and 302 of the sequences SEQ ID NO: 1, 3, and by the nucleotides situated in positions 273 and 299, of the sequence SEQ ID NO: 5.

9. Nucleotide sequence **characterized in that** it corresponds to the bovine *SI* gene represented by SEQ ID NO: 1, or to the following fragments of the *SI* gene:
- any fragment chosen from those of 10 to 300 nucleotides containing the nucleotides situated in positions 82 to 93 of the sequence SEQ ID NO: 1,
- the fragment SEQ ID NO: 7 of 294 nucleotides delimited by the nucleotides situated in positions 9 and 302 of the sequence SEQ ID NO: 1.

10. Nucleotide sequence **characterized in that** it corresponds to the bovine *si* gene represented by SEQ ID NO: 3, or to the following fragments of the *si* gene:
- any fragment chosen from those of 10 to 300 nucleotides containing the nucleotides situated in positions 82 to 93 of the sequence SEQ ID NO: 3,
- the fragment SEQ ID NO: 8 of 294 nucleotides delimited by the nucleotides situated in positions 9 and 302 of the sequence SEQ ID NO: 3.

11. Nucleotide sequence **characterized in that** it corresponds to the bovine *si₁* gene represented by SEQ ID NO: 5, or to the following fragments of the *si₁* gene:
- any fragment chosen from those of 10 to 300 nucleotides containing the nucleotides situated in positions 82 to 93 of the sequence SEQ ID NO: 5,
- the fragment SEQ ID NO: 9 of 291 nucleotides delimited by the nucleotides situated in positions 9 and 299 of the sequence SEQ ID NO: 5.

12. Nucleotide sequence **characterized in that** it comprises:
- the SIL10 nucleotide sequence represented by the following sequence SEQ ID NO: 10:
5' GTTGCTGGAAGGAAGAACAGGATGGATCTG 3'
or any sequence derived from this sequence SEQ ID NO: 10, in particular by suppression and/or substitution and/or addition of one or more nucleotides, said derived sequence being hybridized, like the sequence SEQ ID NO: 10, with all or part of the nucleotide sequence complementary to the nucleotide sequences delimited by the nucleotides situated in positions 9 and 38 of the sequences SEQ ID NO: 1, 3, and 5,
- the SIL8 nucleotide sequence represented by the following sequence SEQ ID NO: 11:
5' CAGTCCCAAGTGCCTGAACACACATGCACC 3'
or any sequence derived from this sequence SEQ ID NO: 11, in particular by suppression and/or substitution and/or addition of one or more nucleotides, said derived sequence being hybridized, like the sequence SEQ ID NO: 11, with all or part of the nucleotide sequences delimited by the nucleotides situated in positions 276 and 302 of the sequences SEQ ID NO: 1, 3, and by the nucleotides situated in positions 273 and 299, of the sequence SEQ ID NO: 5.

13. Primer pairs, each of the two primers comprising, independently of one other, 10 to 30 nucleotides, **characterized in that** they are liable to amplify in a specific way the *SILVER* gene represented by SEQ ID NO: 1, and are chosen in such a manner that one of the two sequences of a primer pair is hybridized with a sequence of 10 to 30 nucleotides comprised in the nucleotide sequence complementary to the sequence delimited by the nucleotides situated in positions 1 and 60 of the nucleotide sequences SEQ ID NO: 1, 3, and 5, whilst the other sequence of this same pair is hybridized with a sequence of 10 to 30 nucleotides comprised between the nucleotide situated in position 94 and the last of the nucleotides of the sequences SEQ ID NO: 1, 3, and 5.

14. Primer pairs for gene amplification according to claim 9, **characterized in that**:
- one of the primers is chosen from the sequences comprising the SIL10 sequence represented by SEQ ID NO: 10, or any sequence derived from the latter, according to claim 12, said primer being advantageously labelled, in particular in a radioactive or fluorescent manner,
- whilst the other primer is chosen from the sequences comprising the SIL8 sequence represented by SEQ ID NO: 11, or any sequence derived from the latter, according to claim 12.

15. Method for the identification of populations or breeds of ruminant mammals such as cattle, sheep and goats, said method being carried out starting with a biological sample collected from the animal, in particular starting with sperm, embryo, blood, milk, hairs, carcass or meat, or other products derived from the latter, and making it possible to check, or even to certify, whether or not the animal from which said biological sample was collected belongs to a population or breed of ruminant mammals, this method comprising:
- a stage of amplification of the number of copies of the different allelic forms of the *SILVER* gene, namely of the *SI,* and/or *si,* and/or *si₁* alleles, and/or of fragments of these allelic forms, specific to a population or breed of specific ruminant mammals, and capable of being present in said biological sample,
- a stage of detection of said allelic forms or fragments of the latter.

16. Identification method according to claim 15, **characterized in that** the stage of amplification of the number of copies of the different allelic forms of the *SILVER* gene, or of the fragments of these allelic forms, is carried out using a primer pair according to claim 13 or 14.

17. Identification method according to claim 15 or 16, **characterized in that**:
- the detection of a genotype comprising *the si* allele in the biological sample studied makes it possible to certify that said sample originates from an animal belonging to the Charolais breed or having at least one ancestor of the Charolais breed,
- the detection of a genotype comprising the *si₁* allele in the biological sample studied makes it possible to certify that said sample originates from an animal belonging to the Simmental breed or having at least one ancestor of the Simmental breed,
- the detection of a genotype comprising the *SI* allele, makes it possible to certify that said sample does not originate from an animal of the Charolais breed.

18. Kit for the implementation of a method according to one of claims 15 to 17, **characterized in that** it comprises at least one primer pair according to claim 13 or 14, and if appropriate the reagents necessary for the implementation of the amplification reaction of the number of copies of the different allelic forms of the *SILVER* gene.

## Patentansprüche

1. Verwendung von Nukleotidsequenzen des bovinen Gens *SILVER,* dargestellt durch SEQ ID NO: 1, das für das bovine Protein SI, dargestellt durch SEQ ID NO: 2, kodiert, und der verschiedenen allelen Formen dieses Gens, oder der Fragmente dieses Gens oder seiner verschiedenen allelen Formen, zum Durchführen eines Verfahrens zum Identifizieren der verschiedenen bovinen Populationen oder Rassen oder der verschiedenen bovinen Herden, die jeweils mehrere bovine Populationen oder Rassen beinhalten.

2. Verwendung von Nukleotidsequenzen nach Anspruch 1 zum Durchführen einer Methode, die es ermöglicht, die, Zugehörigkeit eines Tiers zu einer speziellen bovinen Population oder Rasse oder zu einer speziellen bovinen Herde zu kontrollieren, oder sogar zu zertifizieren, oder die es im Gegensatz dazu ermöglicht, den Ausschluss dieses Tiers aus dieser Population oder Rasse oder aus dieser speziellen Herde zu zertifizieren.

3. Verwendung von Nukleotidsequenzen nach Anspruch 1 oder 2, **dadurch gekennzeichnet, dass** die bovinen Populationen oder Rassen oder bovinen Herden französischen Ursprungs sind.

4. Verwendung nach einem der Ansprüche 1 bis 3:
- der Nukleotidsequenz der allelen Form *si*, dargestellt durch SEQ ID NO: 3, die für das bovine Protein si, dargestellt durch SEQ ID NO: 4, kodiert oder der Fragmente dieser allelen Form, die die Mutation G93A bezogen auf das Gen *SI* umfassen, zum Durchführen einer Methode, die es ermöglicht, die Zugehörigkeit eines Tiers zur Charolais-Rasse zu kontrollieren, oder sogar zu zertifizieren,
- der Nukleotidsequenz der allelen Form *si*, dargestellt durch SEQ ID NO: 5, die für das bovine Protein *si₁*, dargestellt durch SEQ ID NO: 6, kodiert oder der Fragmente dieser allelen Form, die eine Deletion der drei Nukleotide TTC, die an den Positionen 82, 83 und 84 liegen, bezogen auf das Gen *SI* umfasst, zum Durchführen einer Methode, die es ermöglicht, die Zugehörigkeit eines Tiers zur Simmental-Rasse zu kontrollieren, oder sogar zu zertifizieren,
- der Nukleotidsequenz des bovinen Gens *SI*, dargestellt durch SEQ ID NO: 1, oder der Fragmente dieses Gens, zum Durchführen einer Methode, die es ermöglicht, den Ausschluss eines Tiers aus der Charolais-Rasse zu zertifizieren.

5. Verwendung nach einem der Ansprüche 1 bis 4 von Fragmenten der Nukleotidsequenzen der allelen Formen *SI, si* und *si₁,* jeweils dargestellt durch SEQ ID NO: 1, 3 bzw. 5, wobei die Fragmente aus jenen der etwa 10 bis 300 Nukleotide ausgewählt sind, die die Nukleotide enthalten, die an den Positionen 82 bis 93 dieser Sequenzen liegen.

6. Verwendung nach einem der Ansprüche 1 bis 5 von Fragmenten der Nukleotidsequenzen der allelen Formen *SI, si* und *si₁*, wobei die Fragmente aus jenen der 294 Nukleotide ausgewählt sind, die durch die Nukleotide begrenzt sind, die an den Positionen 9 und 302 der Sequenzen SEQ ID NO: 1 und 3 liegen, wobei diese Fragmente jeweils dargestellt werden durch die Sequenzen SEQ ID NO: 7 und SEQ ID NO: 8, und dem Fragment mit 291 Nukleotiden, das durch die Nukleotide begrenzt ist, die an den Positionen 9 und 299 der Sequenz SEQ ID NO: 5 liegen, wobei dieses Fragment durch die Sequenz SEQ ID NO: 9 dargestellt wird.

7. Verwendung von Nukleotidprimern zum Durchführen eines Verfahrens zum Identifizieren der verschiedenen Populationen oder Rassen von wiederkäuenden Säugetieren, wie etwa Rindern, durch Amplifizieren der Kopienanzahl des Gens *SILVER,* oder der verschiedenen allelen Formen dieses Gens oder der Fragmente dieses Gens oder seiner verschiedenen allelen Formen, wie in einem der Ansprüche 1 bis 6 definiert.

8. Verwendung der Nukleotidprimer nach Anspruch 7 in Form von 5'-3'-Primerpaaren, wobei diese Paare so sind, dass:
- der Primer 5' aus dem Primer SIL10 ausgewählt ist, dargestellt durch die folgende Sequenz SEQ ID NO: 10:
5' GTTGCTGGAAGGAAGAACAGGATGGATCTG 3'
oder jeder anderen Sequenz, die von dieser Sequenz SEQ ID NO: 10 abgeleitet ist, insbesondere durch Suppression und/oder Substitution und/oder Addition eines oder mehrerer Nukleotide, wobei die abgeleitete Sequenz, genau wie die Sequenz SEQ ID NO: 10, mit der gesamten oder einem Teil der komplementären Nukleotidsequenz der Nukleotidsequenzen hybridisiert, die durch die Nukleotide begrenzt sind, die an den Positionen 9 und 38 der Sequenzen SEQ ID NO: 1, 3 und 5 liegen,
- der Primer 3' aus dem Primer SIL8 ausgewählt ist, dargestellt durch die folgende Sequenz SEQ ID NO: 11:
5' CAGTCCCAAGTGCCTGAACACACATGCACC 3'
oder jeder anderen Sequenz, die von dieser Sequenz SEQ ID NO: 11 abgeleitet ist, insbesondere durch Suppression und/oder Substitution und/oder Addition eines oder mehrerer Nukleotide, wobei die abgeleitete Sequenz, genau wie die Sequenz SEQ ID NO: 11, mit der gesamten oder einem Teil der Nukleotidsequenzen hybridisiert, die durch die Nukleotide begrenzt ist, die an den Positionen 276 und 302 der Sequenzen SEQ ID NO: 1, 3 und 273 liegen, und durch die Nukleotide, die an den Positionen 273 und 299 der Sequenz SEQ ID NO: 5 liegen,

9. Nukleotidsequenz, **gekennzeichnet durch** das bovine Gen *SI*, dargestellt **durch** SEQ ID NO: 1 oder **durch** die folgenden Fragmente des Gens *SI:*
- jedem Fragment, das aus jenen der etwa 10 bis 300 Nukleotide ausgewählt ist, die die Nukleotide enthalten, die an den Positionen 82 bis 93 der Sequenz SEQ ID NO: 1 liegen,
- dem Fragment SEQ ID NO: 7 mit 294 Nukleotiden, begrenzt **durch** die Nukleotide, die an den Positionen 9 und 302 der Sequenz SEQ ID NO: 1 liegen.

10. Nukleotidsequenz, **gekennzeichnet durch** das bovine Gen *si*, dargestellt **durch** SEQ ID NO: 3 oder **durch** die folgenden Fragmente des Gens *si:*
- jedem Fragment, das aus jenen der etwa 10 bis 300 Nukleotide ausgewählt ist, die die Nukleotide enthalten, die an den Positionen 82 bis 93 der Sequenz SEQ ID NO: 3 liegen,
- dem Fragment SEQ ID NO: 8 mit 294 Nukleotiden, begrenzt **durch** die Nukleotide, die an den Positionen 9 und 302 der Sequenz SEQ ID NO: 3 liegen.

11. Nukleotidsequenz, **gekennzeichnet durch** das bovine Gen *si₁*, dargestellt **durch** SEQ ID NO: 5 oder **durch** die folgenden Fragmente des Gens *si₁*:
- jedem Fragment, das aus jenen der etwa 10 bis 300 Nukleotide ausgewählt ist, die die Nukleotide enthalten, die an den Positionen 82 bis 93 der Sequenz SEQ ID NO: 5 liegen,
- dem Fragment SEQ ID NO: 9 mit 291 Nukleotiden, begrenzt **durch** die Nukleotide, die an den Positionen 9 und 299 der Sequenz SEQ ID NO: 5 liegen.

12. Nukleotidsequenz, **dadurch gekennzeichnet, dass** sie umfasst:
- die Nukleotidsequenz SIL10, dargestellt durch die folgende Sequenz SEQ ID NO: 10:
5' GTTGCTGGAAGGAAGAACAGGATGGATCTG 3'
wobei die Sequenz SEQ ID NO: 10 mit der gesamten oder einem Teil der komplementären Nukleotidsequenz der Nukleotidsequenzen hybridisiert, die durch die Nukleotide begrenzt sind, die an den Positionen 9 und 38 der Sequenzen SEQ ID NO: 1, 3 und 5 liegen,
- die Nukleotidsequenz SIL8, dargestellt durch die folgende Sequenz SEQ ID NO: 11:
5' CAGTCCCAAGTGCCTGAACACACATGCACC 3'
wobei die Sequenz SEQ ID NO: 11 mit der gesamten oder einem Teil der Nukleotidsequenzen hybridisiert, die durch die Nukleotide begrenzt sind, die an den Positionen 276 und 302 der Sequenzen SEQ ID NO: 1, 3 liegen und durch die Nukleotide, die an den Positionen 273 und 299 der Sequenz SEQ ID NO: 5 liegen.

13. Primerpaar, wovon jeder der beiden Primer, unabhängig voneinander, 10 bis 30 Nukleotide umfasst, **dadurch gekennzeichnet, dass** sie in der Lage sind, das bovine Gen *SILVER,* dargestellt durch SEQ ID NO: 1, auf spezifische Weise zu amplifizieren und sie derart ausgewählt sind, dass eine der beiden Sequenzen eines Primerpaars mit einer Sequenz mit etwa 10 bis etwa 30 Nukleotiden hybridisiert, die in der komplementären Nukleotidsequenz der Sequenz enthalten ist, die durch die Nukleotide begrenzt ist, die an den Positionen 1 und 60 der Nukleotidsequenzen SEQ ID NO: 1, 3 und 5 liegen, während die andere Sequenz des selben Paars hybridisiert mit einer Sequenz mit 10 bis 30 Nukleotiden hybridisiert, die zwischen dem Nukleotid, das an Position 94 liegt, und dem letzten der Nukleotide der Sequenzen SEQ ID NO: 1, 3 und 5 enthalten ist.

14. Primerpaar zum gentechnischen Amplifizieren nach Anspruch 10, **dadurch gekennzeichnet, dass**:
- einer der Primer aus den Sequenzen ausgewählt ist, die die Sequenz SIL10, dargestellt durch SEQ ID NO: 10, nach Anspruch 12 umfassen, wobei der Primer vorteilhafterweise, insbesondere radioakiv oder fluoreszierend, markiert ist,
- während der andere Primer aus den Sequenzen ausgewählt ist, die die Sequenz SIL8, dargestellt durch SEQ ID NO: 11, nach Anspruch 12, umfassen.

15. Verfahren zum Identifizieren von Populationen oder Rassen wiederkäuender Säugetiere, wie etwa Rinder, Schafe und Ziegen, wobei das Verfahren ausgehend von einer biologischen Probe ausgeführt wird, die dem Tier entnommen wurde, insbesondere ausgehend von Sperma, Embryo, Blut, Milch, Haaren, Karkasse oder Fleisch oder anderen, von diesen letztgenannten abgeleiteten Produkte, und das es ermöglicht, die Zugehörigkeit oder die Nichtzugehörigkeit des Tiers, dem die biologische Probe entnommen wurde, zu einer Population oder Rasse von wiederkäuenden Säugetieren zu kontrollieren oder sogar zu zertifizieren, wobei dieses Verfahren umfasst:
- einen Schritt des Amplifizierens der Kopienanzahl der verschiedenen allelen Formen des Gens *SILVER,* und zwar der Allele *SI,* dargestellt durch SEQ ID NO: 1, und/oder *si*, dargestellt durch SEQ ID NO: 3, und/oder *si₁*, dargestellt durch SEQ ID NO: 5, und/oder der Fragmente diese allelen Formen, die für eine Population oder Rasse von bestimmten wiederkäuenden Säugetieren spezifisch sind, und in der biologischen probe vorhanden sein können.

16. Verfahren zum Identifizieren nach Anspruch 15, **dadurch gekennzeichnet, dass** der Schritt des Amplifizierens der Kopienanzahl der verschiedenen allelen Formen des Gens *SILVER* oder der Fragmente dieser allelen Formen mit Hilfe eines Primerpaars nach Anspruch 13 oder 14 ausgeführt wird.

17. Verfahren zum Identifizieren nach Anspruch 15 oder 16, **dadurch gekennzeichnet, dass**:
- es der Nachweis eines Genotyps, der das Allel *si* umfasst, in der untersuchten biologischen Probe ermöglicht, zu zertifizieren, dass die Probe von einem Tier stammt, das zur Charolais-Rasse gehört oder mindestens einen Vorfahren der Charolais-Rasse hat,
- es der Nachweis eines Genotyps, der das Allel *si₁* umfasst, in der untersuchten biologischen Probe ermöglicht, zu zertifizieren, dass die Probe von einem Tier stammt, das zur Simmental-Rasse gehört oder mindestens einen Vorfahren der Simmental-Rasse hat,
- es der Nachweis eines Genotyps, der das Allel *SI* umfasst, ermöglicht zu zertifizieren, dass die Probe nicht von einem Tier der Charolais-Rasse stammt.

18. Kit zum Durchführen eines Verfahrens nach einem der Ansprüche 15 bis 17, **dadurch gekennzeichnet, dass** es ein Primerpaar nach Anspruch 13 oder 14 und gegebenenfalls die Reagenzien umfasst, die für das Durchführen der Amplifikationsreaktion der Kopienanzahl der verschiedenen allelen Formen des Gens *SILVER* erforderlich sind.
